(19) Europäisches Patentamt
European Patent Office
Office européen des brevets

(11) Publication number: **0 424 125 A2**

(12) # EUROPEAN PATENT APPLICATION

(21) Application number: **90311401.5**

(22) Date of filing: **17.10.90**

(51) Int. Cl.5: **C07D 239/42**, C07D 495/04, C07D 405/12, A01N 43/54

(30) Priority: **18.10.89 JP 269030/89**
**23.02.90 JP 41145/90**
**27.03.90 JP 75662/90**
**06.04.90 JP 90141/90**
**22.06.90 JP 162643/90**

(43) Date of publication of application:
**24.04.91 Bulletin 91/17**

(84) Designated Contracting States:
**DE ES FR GB IT**

(71) Applicant: **UBE INDUSTRIES, LTD.**
**12-32, Nishihonmachi 1-chome**
**Ube-shi, Yamaguchi-ken(JP)**

(72) Inventor: **Fujii, Katsutoshi, c/o Ube Research Laboratory**
**Ube Industries Ltd., 1978-5, Oaza Kogushi**
**Ube-shi, Yamaguchi-ken(JP)**
Inventor: **Tanaka, Toshinobu, c/o Ube Research Laboratory**
**Ube Industries Ltd., 1978-5, Oaza Kogushi**
**Ube-shi, Yamaguchi-ken(JP)**
Inventor: **Fukuda, Yasuhisa, c/o Ube Research Laboratory**
**Ube Industries Ltd., 1978-5, Oaza Kogushi**
**Ube-shi, Yamaguchi-ken(JP)**

(74) Representative: **Green, Mark Charles et al**
**Urquhart-Dykes & Lord, 91 Wimpole Street**
**London W1M 8AH(GB)**

(54) **Aralkylamine derivatives, preparation method thereof and fungicides containing the same.**

(57) Disclosed is an aralkylamine derivative represented by the following formula (I) and acid addition salt thereof:

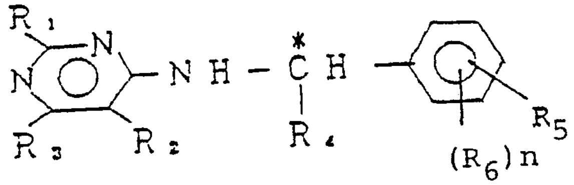

(I)

wherein $R_1$, $R_2$, $R_3$, $R_4$, $R_5$, $R_6$ and n are as described in the description), and preparation method thereof.

EP 0 424 125 A2

# ARALKYLAMINE DERIVATIVES, PREPARATION METHOD THEREOF AND FUNGICIDES CONTAINING THE SAME

## BACKGROUND OF THE INVENTION

This invention relates to aralkylamine derivatives or acid addition salts thereof, processes for producing the same and fungicides containing the same as the active ingredient.

There have been well known a large number of aralkylamine derivatives. For example, Japanese Unexamined Patent Publications Nos. 36666/1984 and 68382/1989 disclose various aminopyrimidine derivatives. These compounds all have insecticidal, acaricidal and fungicidal activities, and are known to be effective against injurious insects such as diamondback moth, aphid, citrus red mite, two-spotted spider mite, etc., and various injurious diseases in agriculture and horticulture such as rice blast, tomato blight, tomato downy mildew, cucumber powdery mildew, etc.

However, although these disclosed compounds have potent activities as insecticidal and acaridical agents, their activities as the fungicidal agent are not sufficient.

## SUMMARY OF THE INVENTION

The object of this invention is to provide aralkylamine derivatives or acid addition salts thereof, processes for producing the same and fungicides containing the same as the active ingredient.

The present inventors have investigated intensively in order to solve the above problem, and consequently found that a novel aralkylamine derivative represented by the following formula have markedly improved fungicidal activity to accomplish the present invention.

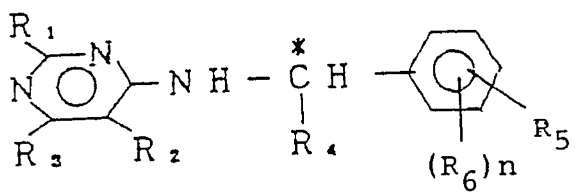

(I)

wherein $R_1$ represents hydrogen atom, a halogen atom, a lower alkyl group, a cycloalkyl group, a lower alkoxy group, a halo-lower alkyl group, an amino group which may be substituted with a lower alkyl group, or a phenyl group which may be substituted;

$R_2$ and $R_3$ each represent hydrogen atom, a halogen atom or a lower alkyl group, or $R_2$ and $R_3$ are fused to the pyrimidine ring together with carbon atoms to which they are bonded to represent a saturated or unsaturated 5- or 6-membered ring which may also have one sulfur atom;

$R_4$ represents hydrogen atom, a lower alkyl group, a cycloalkyl group or a halo-lower alkyl group;

$R_5$ represents $-O-A_1-CN$ or

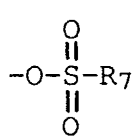

at the 4th position, where $A_1$ represents a lower alkylene group, $R_7$ represents a lower alkyl group, a halo-lower alkyl group; a phenyl group, a benzyl group or a styryl group which may have a substituent; or an amino group which may be substituted with a lower alkyl group; in this case, $R_6$ represents hydrogen atom; or $R_5$ represents

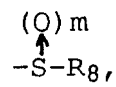

where $R_8$ represents $-CF_2-R_9$ (wherein $R_9$ represents hydrogen atom or a halogen atom) or an alkyl group which is substituted with a lower alkoxy group or a lower alkylthio group; in this case, $R_6$ represents hydrogen atom, a halogen atom, a lower alkyl group, a lower alkoxy group or a halo-lower alkyl group;
or $R_5$ and $R_6$ may form $-O-A_2-O-$ at the 3- and 4-positions, where $A_2$ represents methylene or ethylene group substituted with 1 to 4 halogen atoms;
m represents 0, 1 or 2; and
n represents 1 or 2.

Another embodiment of this invention is a method for producing the compound represented by the formula (I) or acid addition salts thereof which comprises reacting a compound represented by the formula (IIa):

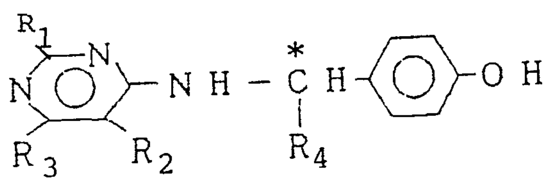

(IIa)

wherein $R_1$, $R_2$, $R_3$ and $R_4$ have the same meanings as defined above, with at least one of a compound represented by the formula (IIIa):
$X-A_1-CN$ (IIIa)
and a compound represented by the formula (IIIb):

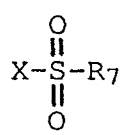

(IIIb)

wherein $A_1$ and $R_7$ have the same meaning as defined above, and X represents an eliminable group.

A further embodiment of this invention is a method for producing the aralkylamine derivatives or acid addition salts thereof represented by the formula (I) which comprises reacting a compound represented by the formula (IIb):

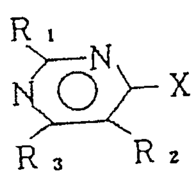

(IIb)

wherein $R_1$, $R_2$, $R_3$ and X have the same meanings as defined above, with a compound represented by the formula (IIIc):

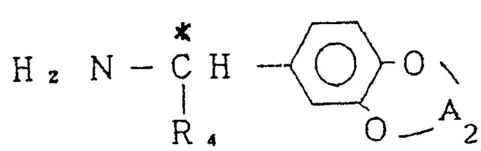

(IIIc)

wherein $R_4$ and $A_2$ have the same meaning as defined above.

Yet further embodiment of this invention is a method for producing aralkylamine derivatives or acid addition salts thereof represented by the formula (I) which comprises: reacting a compound represented by the formula (IIc):

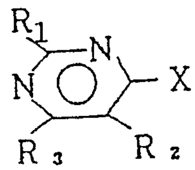

(IIc)

(wherein $R_1$, $R_2$, $R_3$ and X have the same meanings as defined above), with a compound represented by the formula (IIId):

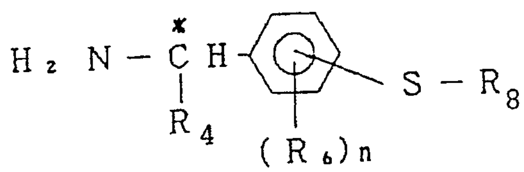

(IIId)

(wherein $R_4$, $R_6$, $R_8$ and n have the same meaning as defined above) to obtain a compound represented by the formula (I-d'):

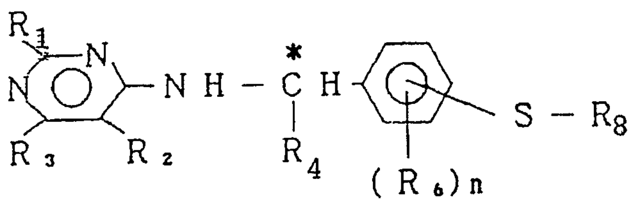

(I-d')

(wherein $R_1$, $R_2$ $R_3$, $R_4$, $R_6$, $R_8$ and n have the same meanings as defined above)
and then optionally oxidizing the compound represented by the formula (I-d') with a peroxide to obtain a compound represented by the formula (I-d"):

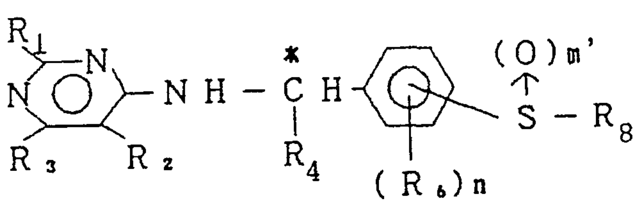

(I-d")

(wherein $R_1$, $R_2$ $R_3$, $R_4$, $R_6$, $R_8$ and n have the same meanings as defined above, and m' represents 1 or 2).

Another embodiment of this invention relates to fungicides containing the aralkylamine derivatives or acid addition salts thereof as an active ingredient.

A still further embodiment of this invention is benzene derivatives having a cyclic ether substituted with fluorine atom or atoms and processes for producing the same shown below.

(1) An amine represented by the formula (VIII):

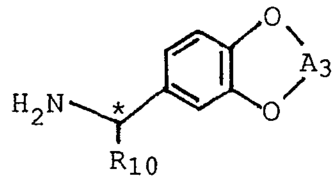

(VIII)

(wherein $R_{10}$ represents a lower alkyl group or a cycloalkyl group, and $A_3$ represents methylene group or ethylene group substituted with 1 to 4 fluorine atoms).

(2) An oxime, which is an intermediate in synthesis of the amine represented by the formula (VIII), represented by the formula (IX):

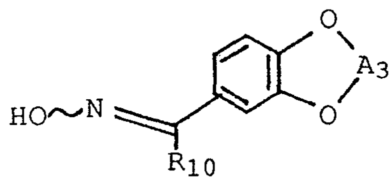

(IX)

(wherein $R_{10}$ and $A_3$ have the same meanings as defined above).

(3) A ketone, which is an intermediate in synthesis of the amine represented by the formula (VIII), represented by the formula (X):

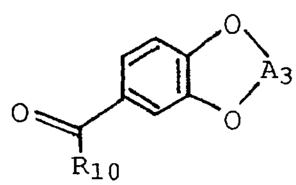

(X)

(wherein $R_{10}$ and $A_3$ have the same meaning as defined above).

(4) A method for producing the amine represented by the formula (VIII) which comprises carrying out hydrogen reduction of an oxime represented by the formula (IX).

(5) A method for producing the oxime represented by the formula (IX) which comprises reacting the ketone represented by the formula (X) with a salt of hydroxyamine in the presence of a base.

(6) A method for producing the ketone represented by the formula (X) which comprises reacting a compound represented by the formula (XI):

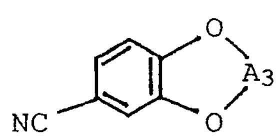

(XI)

(wherein $A_3$ has the same meaning as defined above) with a compound represented by the formula (XII):

$R_{10}MgX_3$ (XII)

(wherein $R_{10}$ has the same meaning as defined above, and $X_3$ represents a halogen atom).

(7) A method for producing the ketone represented by the formula (XV):

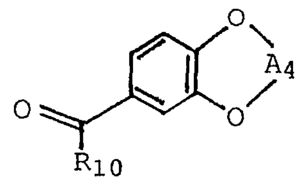

(XV)

(wherein $R_{10}$ has the same meaning as defined above, and $A_4$ represents ethylene group substituted with 2 to 4 fluorine atoms),
which comprises reacting a compound represented by the formula (XIII):

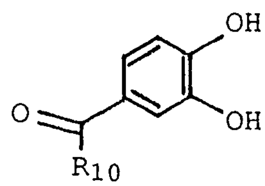

(XIII)

(wherein $A_{10}$ has the same meaning as defined above) with a compound represented by the formula (XIV):

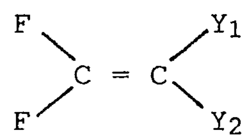

(XIV)

(wherein $Y_2$ represents a halogen atom, and $Y_1$ represents hydrogen atom or a halogen atom).

## DESCRIPTION OF THE PREFERRED EMBODIMENTS

The aralkylamine derivatives which is a desired compound of this invention and the compounds of the formulae (IIa), (IIb), (IIc), (IIIa), (IIIb), (IIIc) and (IIId) which are starting materials thereof will be described below.

As $R_1$, hydrogen atom, a halogen atom, a lower alkyl group, a cycloalkyl group, a lower alkoxy group, a lower haloalkyl group, an amino group which may be substituted with a lower alkyl group, or a phenyl group which may be substituted, and the like may be included. Preferred is hydrogen atom.

As $R_2$, hydrogen atom, a halogen atom, a lower alkyl group and the like may be included. Among them, preferred is a halogen atom such as fluorine atom, chlorine atom, bromine atom, iodine atom, etc., and further preferred is chlorine atom.

As $R_3$, hydrogen atom, a halogen atom, a lower alkyl group and the like may be included. Among them, preferred is a lower alkyl group such as a straight- or branched-chain alkyl group having 1 to 5 carbon atoms, and further preferred is methyl group, ethyl group and isopropyl group.

Alternatively, $R_2$ and $R_3$ are fused to the pyrimidine ring together with carbon atoms to which they are bonded to represent a saturated or unsaturated 5- or 6-membered ring which may also have one sulfur atom. Preferably, these form a benzene ring or a thiophene ring.

As $R_4$, hydrogen atom, a lower alkyl group, a cycloalkyl group, a halo-lower alkyl group such as an alkyl group having 1 to 2 carbon atoms substituted with 1 to 3 halogen atoms (e.g. chloromethyl group, 1-chloroethyl group, 2-chloroethyl group, monofluoromethyl group, difluoromethyl group, trifluoromethyl group, 1-fluoroethyl group, 2-fluoroethyl group, 2,2,2-trifluoroethyl group, etc.) and the like may be included. Among them, preferred is hydrogen atom, a lower alkyl group such as a straight- or branched-chain alkyl group having 1 to 8, preferably 1 to 5, carbon atoms, a cycloalkyl group such as those having 3 to 8 carbon atoms, and further preferred is, as the lower alkyl group, a straight- or branched-chain alkyl group having 1

to 3 carbon atoms such as methyl group, ethyl group, isopropyl group, etc. and, as the cycloalkyl group, those having 3 to 6 carbon atoms such as cyclopropyl group, cyclopentyl group, cyclohexyl group, etc., more preferably cyclopropyl group.

When $R_5$ represents $-O-A_1-CN$ or

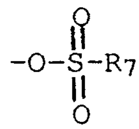

at the 4-position, $R_6$ represents hydrogen atom. When $R_5$ represents

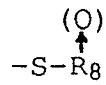 at

the 3- or 4-position, $R_6$ represents hydrogen atom, a halogen atom, a lower alkyl group, a lower alkoxy group, a halo-lower alkyl group and the like, preferably hydrogen atom.

m represents 0, 1 or 2. $m'$ represents 1 or 2. n represents 1 or 2, preferably 1.

In the preparation method of this invention, the eliminable group X, which is not particularly limited, may include, for example, a halogen atom such as chlorine atom, bromine atom, iodine atom, etc., an alkylthio group such as methylthio, ethylthio, propylthio, butylthio, etc., an alkanesulfonyloxy group which may be substituted with a halogen atom such as methanesulfonyloxy, ethanesulfonyloxy, trifluoromethanesulfonyloxy, etc., an arylsulfonyloxy group such as benzenesulfonyloxy, p-toluenesulfonyloxy, etc., hydroxy group and the like. Preferred is halogen atom, and further preferred is chlorine atom.

When $R_5$ denotes

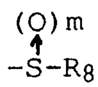,

the substitution position of

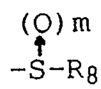

may be the 3- or 4-position, preferably the 4-position.

$R_7$ may include a lower alkyl group, a halo-lower alkyl group; a phenyl group, a benzyl group or a styryl group which may have a substituent; an amino group which may be substituted with a lower alkyl group and the like. The lower alkyl group may include a straight- or branched-chain alkyl group having 1 to 5 carbon atoms such as methyl, ethyl, propyl, isopropyl, butyl, sec-butyl, t-butyl, pentyl, isopentyl, neopentyl, etc. The halo-lower alkyl group may include an alkyl group, having 1 to 3 carbon atoms, substituted with 1 to 5 halogen atoms, such as monofluoromethyl, difluoromethyl, trifluoromethyl, monochloromethyl, trichloromethyl, 2-fluoroethyl, 2,2,2-trifluoroethyl, 1,1,2,2,2-pentafluoroethyl, 3,3,3-trifluoropropyl, 3-chloropropyl, etc. The phenyl group which may have a substituent may include a phenyl group which may be substituted with a lower alkyl, a halogen atom or trifluoromethyl, such as phenyl, 4-chlorophenyl, 4-fluorophenyl, 4-methylphenyl, 4-trifluoromethylphenyl, etc. The amino group which may be substituted with a lower alkyl group may include an unsubstituted amino group and an amino groups substituted with 1 or 2 alkyl groups having 1 to 5 carbon atoms, such as amino, monomethylamino, dimethylamino, monoethylamino, diethylamino, monopropylamino, dipropylamino, monobutylamino, dibutylamino, monopentylamino, dipentylamino, etc.

$R_8$ may include $-CF_2-R_9$, a lower alkyl group, a lower alkyl group substituted with a lower alkylthio group or a lower alkoxy group and the like. Preferred is a lower alkyl group such as methyl group, ethyl group, etc. and a lower alkyl group substituted with a lower alkoxy group such as 2-ethoxyethyl group, etc.

$R_9$ may include hydrogen atom and a halogen atom, preferably hydrogen atom.

The lower alkylene group as $A_1$ may include an alkylene group having 1 to 5 carbon atoms such as methylene, 1-methylmethylene, ethylene, 1-methylethylene, 2-methylethylene, propylene, pentylene, etc., preferably methylene group, 1-methylmethylene group, ethylene group or propylene group, further preferably methylene group, ethylene group or propylene group.

$A_2$ may include methylene group substituted with 1 to 2 halogen atoms, ethylene group substituted with 1 to 4 halogen atoms and the like. Preferred are methylene group substituted with 1 to 2 fluorine atoms such as monofluoromethylene group, difluoromethylene group, etc., ethylene group substituted with 1 to 4 fluorine atoms such as monof luoroethylene group, difluoroethylene group, trifluoroethylene group, tetrafluoroethylene group, etc. and the like.

As the pyrimidinyl group on which $R_2$ and $R_3$ are substituted, the following are enumerated.

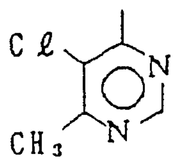
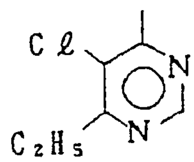
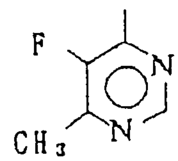
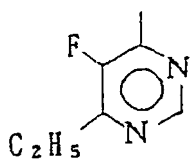
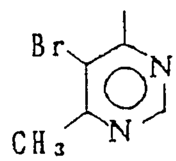
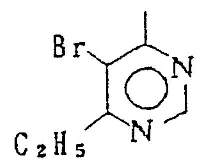
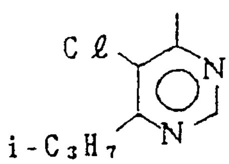
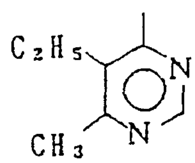
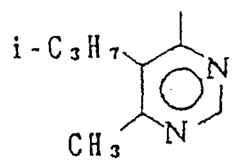
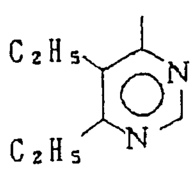
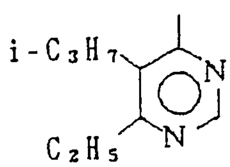
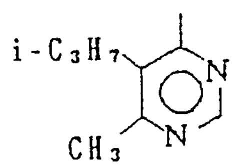
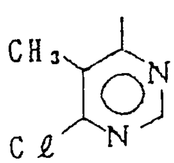
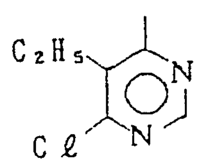
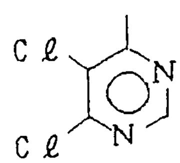
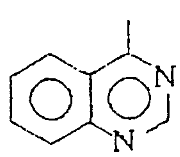
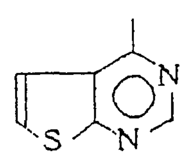

Among them, preferred are

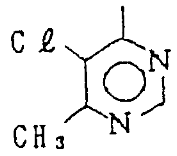
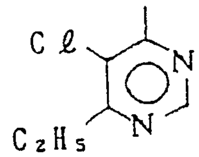
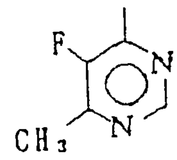

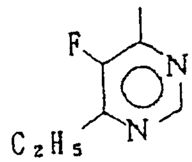
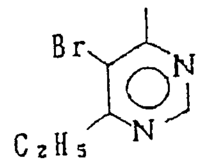
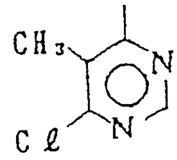

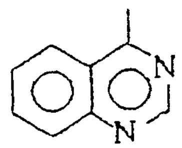 and 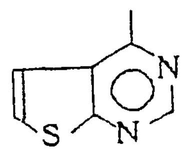 .

As will be understood from the formula (I), the compound of this invention contains an amino group, and thus forms an acid addition salt easily. Such a salt may also be included in the present invention.

The acid which forms the acid addition salt may include, for example, inorganic acids such as hydrochloric acid, hydrobromic acid, nitric acid, sulfuric acid, phosphoric acid, etc.; carboxylic acids such as formic acid, oxalic acid, fumaric acid, adipic acid, stearic acid, oleic acid, aconitic acid, etc.; organic sulfonic acids such as methanesulfonic acid, benzenesulfonic acid, p-toluenesulfonic acid, etc.; saccharin, and the like.

In the formula (I), when the carbon atom marked by * is an asymmetric atom, respective optical isomers and racemic compounds or a mixture thereof are all included in the present invention.

Among the desired compound (I) of the present invention, preparation method of the compound represented by the formula (Ia) will be described below.

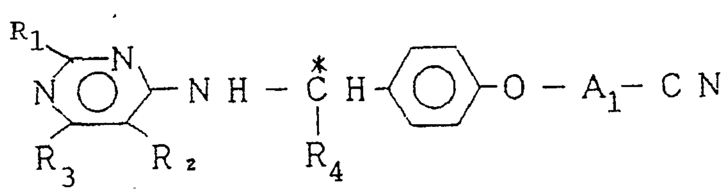 (Ia)

The material compound (IIa) used in the present invention can be easily prepared by carrying out the following process.

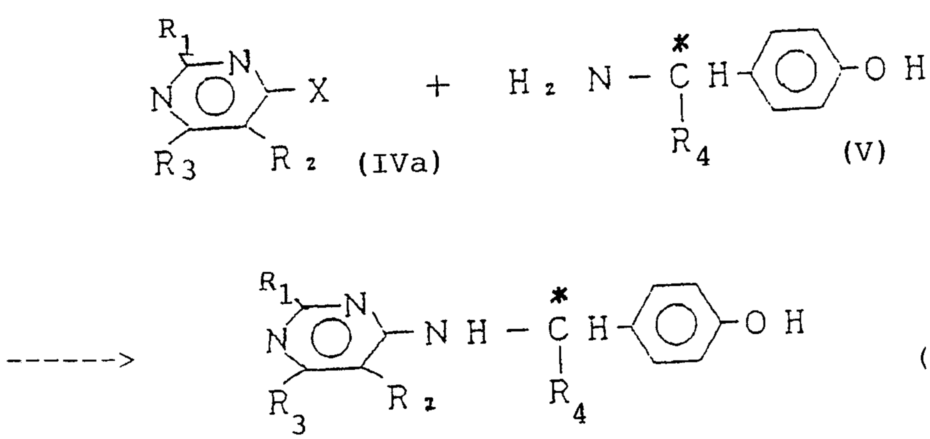
(IVa) (V)
(IIa)

(wherein $R_1$, $R_2$, $R_3$, $R_4$ and X have the same meanings as described above)

The compound of the formula (V) used in the preparation method of the compound of the formula (IIa) can be prepared easily by carrying out the scheme as shown below according to the process described in, for example, Journal of American Chemical Society (J.A.C.S) 79 , 1455 (1957), etc.

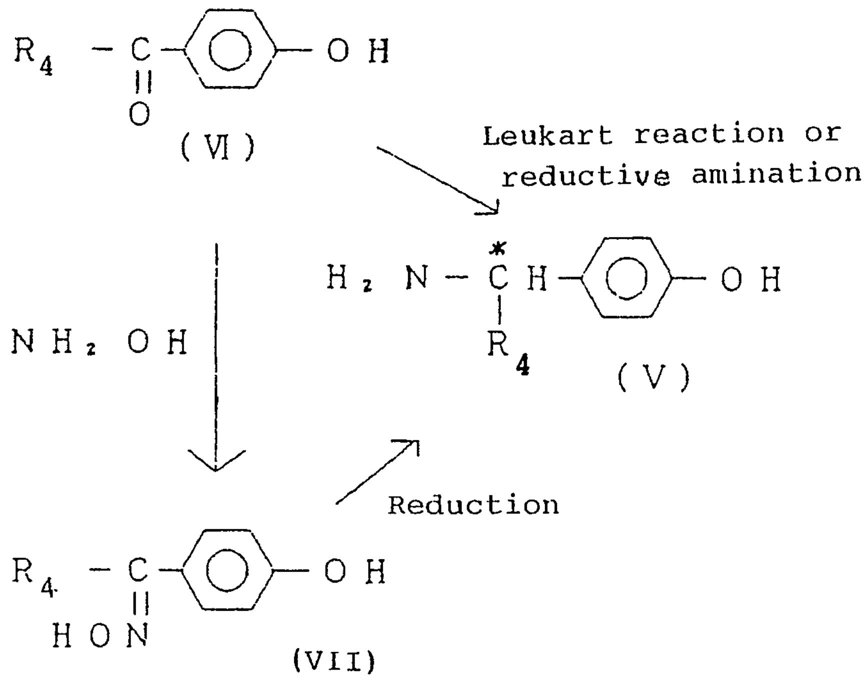


(wherein $R_4$ has the same meaning as described above)

The desired compound (Ia) of the present invention can be prepared by, for example, carrying out the following scheme.

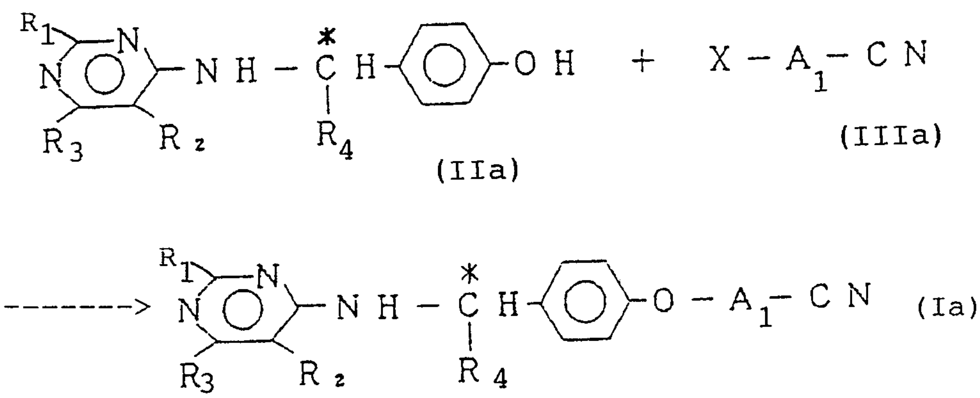


(wherein $R_1$, $R_2$, $R_3$, $R_4$, $A_1$ and X have the same meanings as the above)

Usually, the desired compound (Ia) is preferably produced by reacting the material compound (IIa) and the material compound (IIIa) in a solvent in the presence of a base, but may be obtained by the reaction without adding a base, or may be obtained by reacting the material compounds (IIa) and (IIIa) in the absence of a solvent by heating and dissolving them.

As the solvent, there is no particular limitation so far as it does not take part in the reaction directly, included are, for example, chlorinated or unchlorinated aromatic, aliphatic or alicyclic hydrocarbons such as benzene, toluene, xylene, methyl naphthalene, petroleum ether, ligroin, hexane, chlorobenzene, dichlorobenzene, methylene chloride, chloroform, dichloroethane, trichloroethane, cyclohexane, etc.; ethers such as diethyl ether, tetrahydrofuran, dioxane, etc.; ketones such as acetone, methyl ethyl ketone, etc.; alcohols such as methanol, ethanol, ethylene glycol, etc. or their hydrous products; amides such as N,N-dimethylformamide (DMF), N,N-dimethylacetamide, etc.; organic bases such as triethylamine, pyridine, N,N-diethylaniline, etc.; 1,3-dimethyl-2-imidazolidinone (DMI); dimethylsulfoxide (DMSO); and mixtures of the above-mentioned solvents.

The base may include, for example, organic bases such as triethylamine, pyridine, N,N-diethylaniline, etc.; alkali metal alkoxides such as sodium methoxide, sodium ethoxide, etc.; inorganic bases such as sodium amide, sodium hydroxide, potassium hydroxide, potassium carbonate, sodium carbonate, sodium hydride, etc. For accelerating the reaction ratio, 4-(N,N-dimethylamino) pyridine is preferably added as a catalyst.

The preparation method of the desired compound (Ia) is carried out at the concentration of substrate of 5 to 100 %.

In the preparation method, the ratio of the material compounds (IIa) and (IIIa) is usually 0.5 to 1.5 mol, preferably 0.5 to 1.0 mol, of the material compound (IIIa) per mol of the material compound (IIa).

The reaction temperature is not particularly limited so far as the reaction is carried out below the boiling point of the solvent to be used, but usually at a room temperature or higher, and preferably under heating around the boiling point of the solvent to shorten the reaction time.

The reaction time may vary depending on the above-mentioned concentration and temperature, but be usually 2 to 10 hours.

The desired compound (Ia) can be appropriately purified by conventional methods such as recrystallization, various chromatographies, etc. The acid addition salts of the compound can be easily obtained by, for example, the steps of introducing an acid into the reaction solution after the reaction is completed, and removing the solvent by evaporation under reduced pressure, or by the steps of dissolving the desired compound (Ia) in a solvent, introducing an acid thereinto and filtrating the settled crystal or removing the solvent by evaporation under reduced pressure.

Among the compounds (I) of the present invention, the compound represented by the following formula (Ib) can be easily prepared by the method well know per se as shown below.

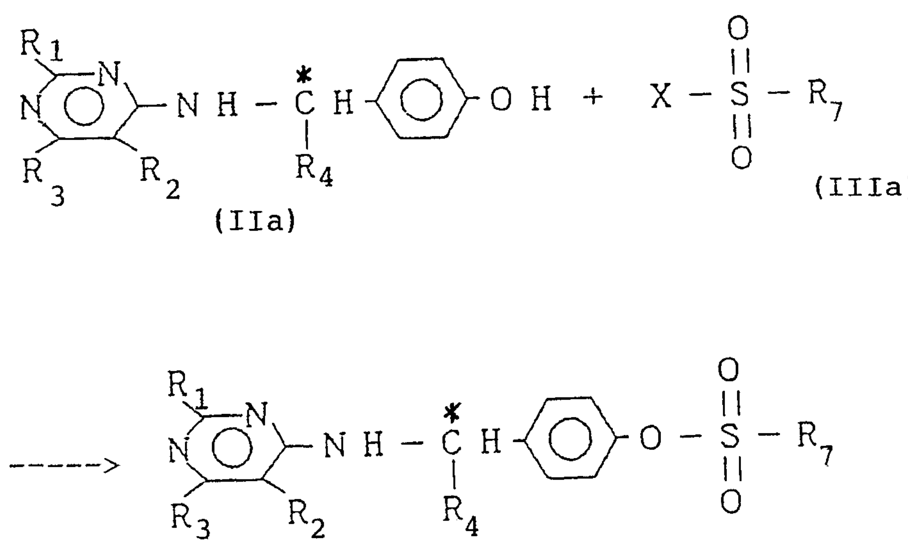

(IIa) (IIIa)

(Ib)

(wherein $R_1$, $R_2$, $R_3$, $R_4$ and $R_7$ have the same meanings as the above, and X denotes an eliminable group)

The above reaction is well know per se . The eliminable group X is not limited at all and may include, for example, halogen atoms such as chlorine, bromine or iodide; alkylthio groups such as methylthio, ethylthio, propylthio, butylthio, etc.; alkanesulfonyloxy groups such as methanesulfonyloxy, ethanesulfonyloxy, trifluoromethanesulfonyloxy, etc.; arylsulfonyloxy groups such as benzenesulfonyloxy, p-toluenesulfonyloxy, etc.; and hydroxy group, and the like.

In the above reaction, the compound H-X is released. In order to capture the compound H-X and proceed the reaction smoothly, the desired compound (Ib) may preferably be prepared by reacting the material compounds (IIa) and (IIIb) in a solvent in the presence of a base. However, the reaction may be carried out without addition of the base. The desired compound (Ib) may be prepared by heating and dissolving the material compounds (IIa) and (IIIb) in the absence of a solvent to proceed the reaction.

As the solvent and base, those enumerated in the preparation method of the desired compound (Ia) can be used.

The reaction temperature is not particularly limited, but usually at a room temperature or higher and at the boiling point or lower of the solvent to be used. It is preferred to apply heat in the reaction for

shortening the reaction time.

The intermediate (IIa) used in the present invention can be prepared by the method which is well known per se as shown below.

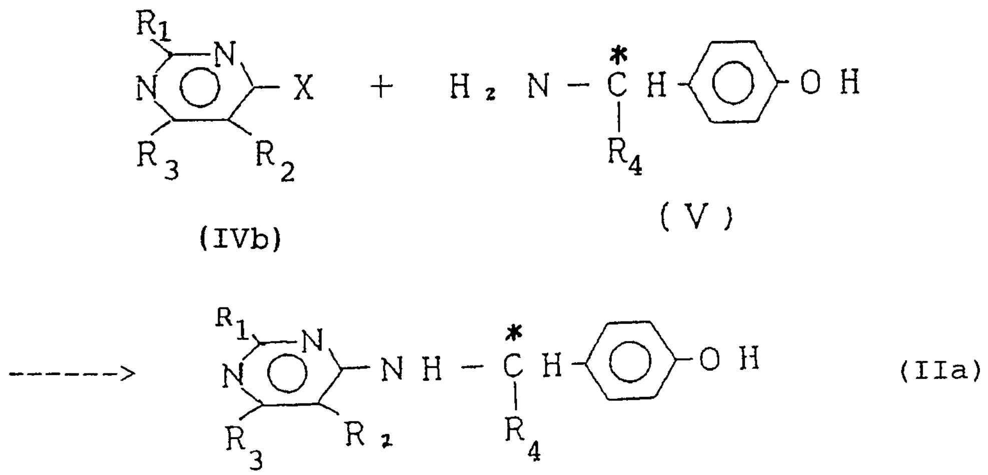

(IVb) (V) (IIa)

(wherein $R_1$, $R_2$, $R_3$, $R_4$ and X have the same meanings as described above)

As the solvent, base, etc. used in the process, those described in the preparation method of the compound (Ia) can be appropriately used.

In the above preparation method, the compound of the formula (V) used as a starting material can also be produced according to conventional process well known per se as shown below.

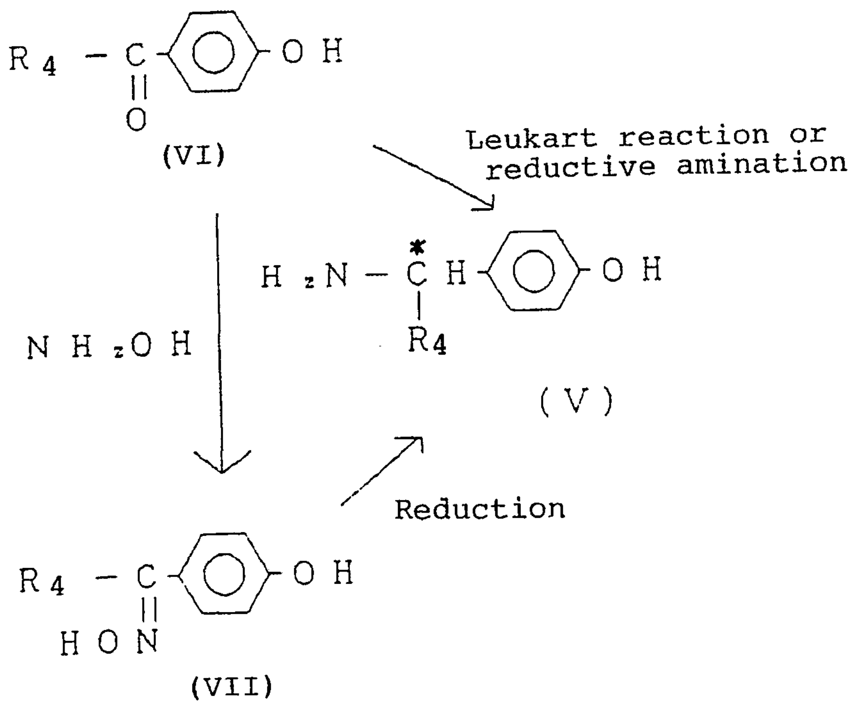


(VI) (V) (VII)

(wherein $R_4$ has the same meaning as described above)

The compound of the formula (Ib), which is obtained by the above respective processes, can be appropriately purified by conventional methods such as recrystallization, various chromatographies, etc.

The acid addition salts of the compound can be easily obtained by, for example, the steps of introducing an acid into the reaction solution after the reaction is completed, and removing the solvent by evaporation under reduced pressure, or by the steps of dissolving the desired compound (Ib) in a solvent, introducing an acid thereinto and filtrating the settled crystal or removing the solvent by evaporation under

reduced pressure.

Among the desired compound (I) of the present invention, the compound of the formula (Ic) as shown below can be prepared by, for example, carrying out the reaction scheme as shown below.

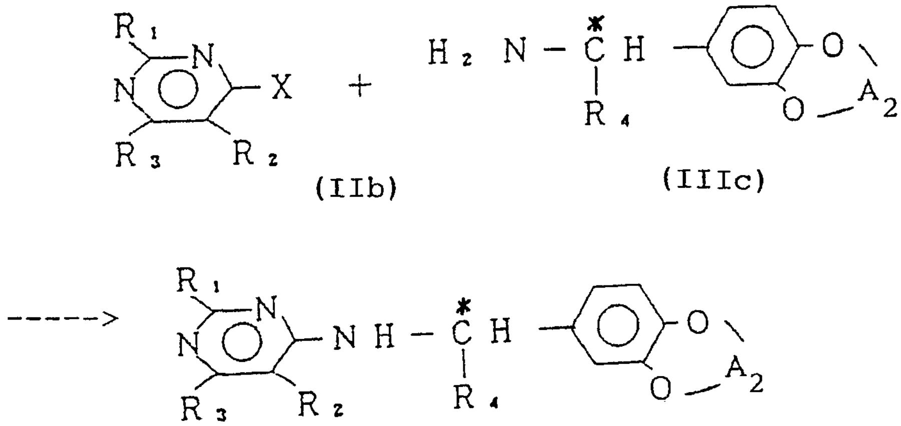

(IIb) (IIIc)

(Ic)

(wherein $R_1$, $R_2$, $R_3$, $R_4$, $A_2$ and X have the same meanings as described above)

The material compound (IIb) used in the present invention can be easily prepared by, for example, carrying out the reaction as shown below according to the method described in Journal of Chemical Society (J.C.S), p.p. 3478 - 3481 (1955).

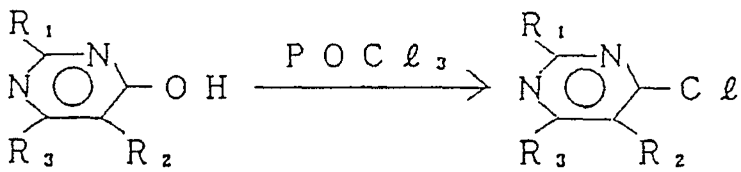


(wherein $R_1$, $R_2$ and $R_3$ have the same meanings as described above)

Usually, the desired compound (Ic) is preferably produced by reacting the material compound (IIb) and the material compound (IIIc) in a solvent in the presence of a base, but may be obtained by the reaction without adding a base, or may be obtained by reacting the material compounds (IIb) and (IIIc) in the absence of a solvent by heating and dissolving them.

As the solvent and base, those used in the preparation method of the desired compound (Ia) as described above may be used. Also, in order to increase the reaction rate, it is preferred to add 4-(N,N-dimethylamino)pyridine as a catalyst.

The reaction temperature is not particularly limited, but usually within the temperature range from a room temperature to the boiling point of the solvent to be used. The reaction time can be shortened by heating within the temperature range at the boiling point or lower.

The reaction time may vary depending on the concentration and temperature as described above, but usually within 3 to 8 hours.

The desired compound (Ic) prepared as described above can be appropriately purified by conventional methods such as recrystallization, various chromatographies, etc.

The acid addition salts of the compound can be easily obtained by, for example, the steps of introducing an acid into the reaction solution after the reaction is completed, and removing the solvent by evaporation under reduced pressure, or by the steps of dissolving the desired compound (Ic) in a solvent, introducing an acid thereinto and filtrating the settled crystal or removing the solvent by evaporation under reduced pressure.

Among the desired compound (I) of the present invention, the compound of the formula (Id) including the formulae (Id′) and (I″) as shown below can be prepared by, for example, carrying out the reaction scheme as shown below.

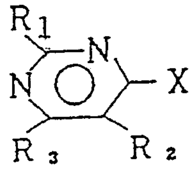

(IIc)

+

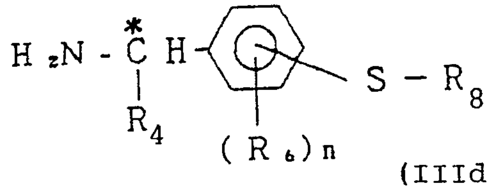

(IIId)

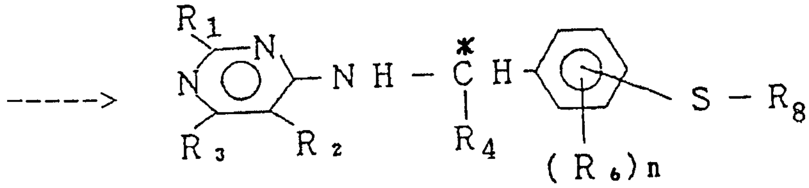

(Id')

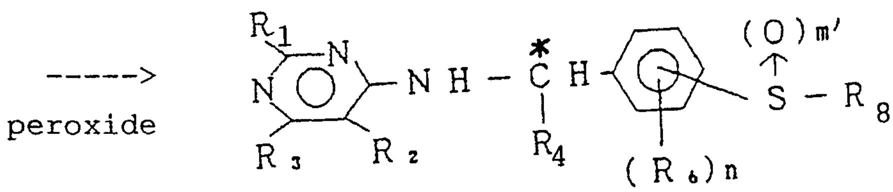


(Id")

(wherein $R_1$, $R_2$, $R_3$, $R_4$, $R_6$, $R_8$, m′, n and X have the same meanings as described above)

The material compound (IIc) used in the present invention can be easily prepared by, for example, carrying out the reaction as shown below according to the method described in Journal of Chemical Society (J.C.S), p.p. 3478 - 3481 (1955).

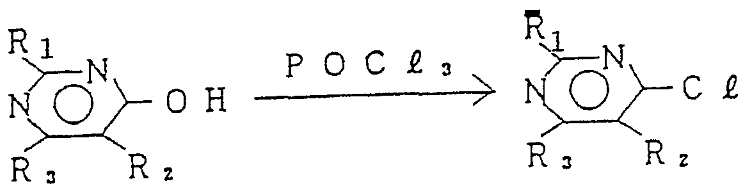


(wherein $R_1$, $R_2$ and $R_3$ have the same meanings as described above)

The material compound (IIId) used in the present invention can be easily prepared by, for example, carrying out the reaction as shown below according to the method described in Journal of American Chemical Society (J.A.C.S), 79 , 1455 (1957), etc.

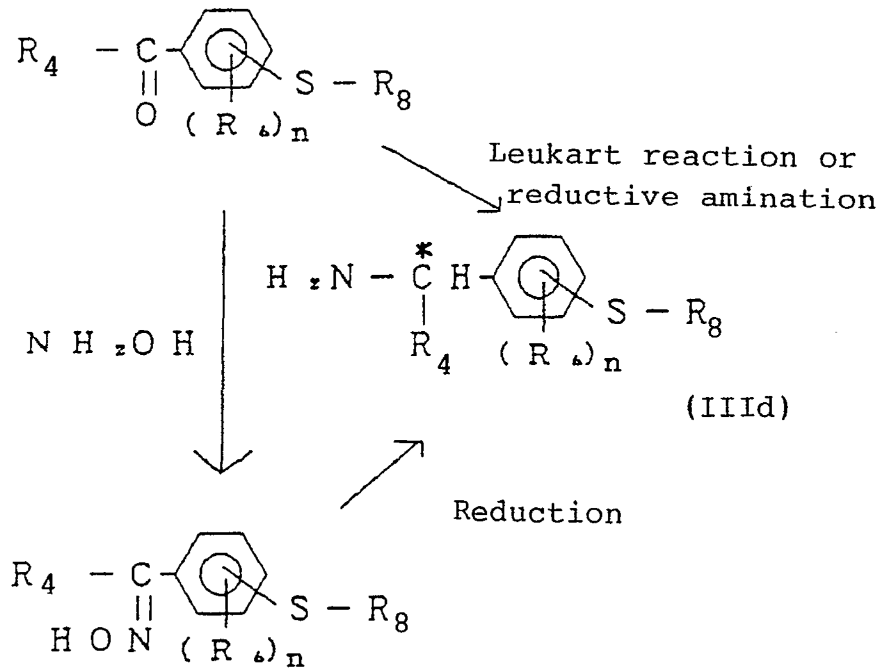


(IIId)

(wherein $R_4$, $R_6$, $R_8$ and n have the same meanings as the above)

The peroxide, which is not particularly limited, may include, for example, hydrogen peroxide, m-chloro perbenzoic acid, sodium meta periodate, potassium permanganate, sodium hypochlorite, etc.

Preparation method of the desired compound (Id′)

Usually, the desired compound (Id″) may preferably be produced by reacting the material compound (IIc) and the material compound (IIId) in a solvent in the presence of a base, but may be obtained by the reaction without adding a base, or may be obtained by reacting the material compounds (IIc) and (IIId) in the absence of a solvent by heating and dissolving them.

As the solvent and base, those used in the preparation method of the desired compound (Ia) as described above are used. Also, in order to increase the reaction rate, it is preferred to add 4-(N,N-dimethylamino)pyridine as a catalyst.

The reaction temperature is not particularly limited, but usually within the temperature range from a room temperature up to the boiling point of the solvent to be used. The reaction time can be shortened by heating within the temperature range at the boiling point or lower of the solvent.

The reaction time may vary depending on the concentration and temperature as described above, but usually within 3 to 8 hours.

Preparation method of the desired compound (Id″)

The desired compound (Id″) can be produced by reacting the desired compound (Id′) and a peroxide in a solvent.

As the solvent, there is no particular limitation so far as the solvent does not take part in the reaction directly, but included are chlorinated aliphatic hydrocarbons such as chloroform, dichloromethane, etc.; alcohols such as methanol, ethanol, etc.; ketones such as acetone, methyl ethyl ketone, etc.; cyclic ethers such as tetrahydrofurane, dioxane, etc.; aliphatic carboxylic acids such as acetic acid, propionic acid, etc.; water and mixtures of the above solvents.

The ratio of the compound (Id′) to the peroxides is usually 0.5 to 3 mol, preferably 1 to 2.2 mol per mol of the compound (Id′).

The reaction temperature is not particularly limited so far as the solvent is used in the temperature range between -10 °C and the boiling point of the solvent to be used or lower, but usually preferably in the range of -10 °C to a room temperature.

The reaction time may vary depending on the concentration and temperature as described above, but

usually within 1 to 8 hours.

The desired compound (Id′) and (Id″) prepared as described above can be appropriately purified by conventional methods such as recrystallization, various chromatographies, etc.

The acid addition salts of the compound can be easily obtained by, for example, the steps of introducing an acid into the reaction solution after the reaction is completed, and removing the solvent by evaporation under reduced pressure, or by the steps of dissolving the desired compound (Id) in a solvent, introducing an acid thereinto and filtrating the settled crystal or removing the solvent by evaporation under reduced pressure.

The benzene derivatives, represented by the formula (VIII), having a cyclic ether substituted with fluorine atom or atoms is an effective intermediate for the compound (Ic) of this invention shown below:

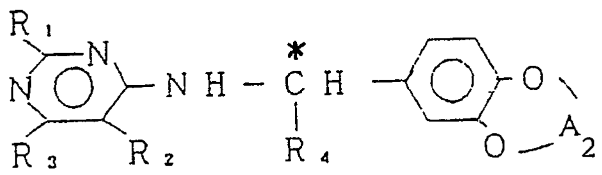

(Ic)

The present inventors have found that the above-mentioned benzene derivatives are effective intermediate for pesticides and pharmaceuticals, and investigated intensively preparation methods of them, and resultingly found the method shown below to complete the present invention.

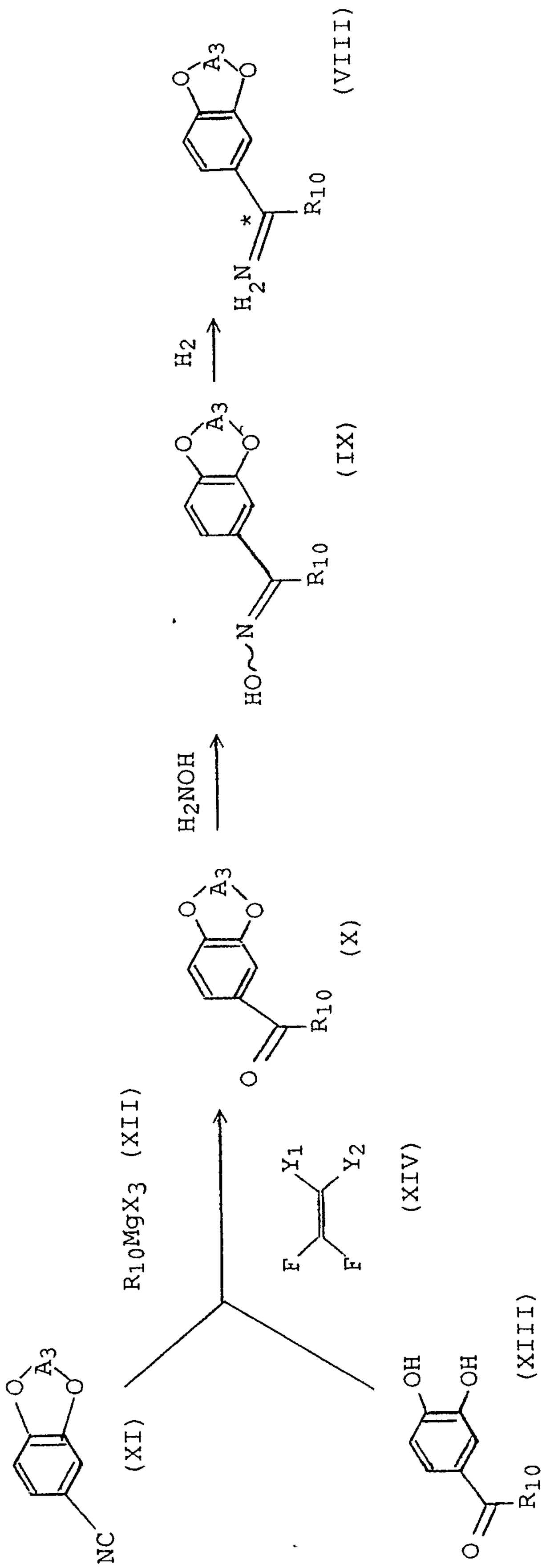


(VIII)

(IX)

(X)

(XI)

(XIII)

(XIV)

(wherein $R_{10}$ represents a lower alkyl group or a cycloalkyl group, $A_3$ represents methylene group or ethylene group substituted with 1 to 4 fluorine atoms, and $X_3$ represents a halogen atom).

In the novel benzylamine derivative of the formula (VIII) and its starting material of the formula (IX) and (X) of the present invention, $R_9$ represents hydrogen atom, a lower alkyl group, a cycloalkyl group, a halo-lower alkyl group having 1 to 2 carbon atoms such as monofluromethyl group, dif luoromethyl group, trifluoromethyl group, 2-fluoroethyl group, 2,2,2-trifluoroethyl group, etc. Among them, preferred are a lower alkyl group such as a straight- or branched-chain alkyl group having 1 to carbon atoms, a cycloalkyl group such as those having 3 to 8 carbon atoms, and further preferred are a substituted or unsubstituted lower alkyl group having 1 to 3 carbon atoms such as methyl group, ethyl group, isopropyl group, etc., a cycloalkyl group having 3 to 6 carbon atoms such as cyclopropyl group, cyclopentyl group, cyclohexyl group, etc.

$A_3$ represents methylene group substituted with 1 to 2 fluorine atoms such as monofluoromethylene group, difluoromethylene group, etc., and ethylene group substituted with 1 to 4 fluorine atoms such as monofluoroethylene group, difluoroethylene group, trifluoroethylene group, tetrafluoroethylene group, etc.

The benzylamine derivatives of the formula (VIII) of this invention can be synthesized by hydrogenation of the oxime of the formula (IX) in a solvent in the presence of a catalyst. The solvent, which is not particularly limited provided that it does not take a part directly in the reaction, may include, for example, alcohols such as methanol, ethanol, isopropanol, ethyleneglycol, etc., hydrocarbons such as toluene, xylene, benzene, etc. and the like. The reaction temperature is not particularly limited, but usually 20 to 150 °C. The reaction is usually carried out under a hydrogen pressure between a normal pressure and 60 kg/cm$^2$. A hydrogenation catalyst used in the reaction is not particularly limited, but preferably includes Ra-Ni, reduced Ni, Pd-C, etc. The reaction time is usually 1 to 5 hours.

The compound of the present invention is very effective for barley powdery mildew and wheat brown rust, and otherwise useful as the fungicide for agriculture and horticulture such as cucumber downy mildew, tomato blight, rice blast, etc.

Also, the compound of the present invention is effective for agricultural and horticultural injurious insects such as planthoppers, leafhoppers, aphids, whiteflies, diamondback moth, etc., and otherwise also exhibits activity against citrus red mite, two-spotted spider mite, etc.

Thus, the uses, application fields of the compound of the present invention are very wide, high in activity and can be provided for practical application in various dosage forms.

The fungicide of the present invention contains one kind or two or more kinds of the compound of the formula (I) as the active ingredient.

The compound of the formula (I) may be also used as such, but generally formulated with carrier, surfactant, dispersing agent and auxiliary agent to prepare a composition such as powder, emulsion, fine particle, granule, wettable agent or oily suspension, aerosol, etc. before use.

Examples of preferable carrier may include solid carriers such as talc, bentonite, clay, kaolin, diatomaceous earth, white carbon, vermicullite, slaked lime, siliceous sand, ammonium sulfate and urea; liquid carriers including hydrocarbons such as kerosine and mineral oil, aromatic hydrocarbons such aS benzene, toluene and xylene, chlorinated hydrocarbons such as chloroform and carbon tetrachloride, ethers such as dioxane and tetrahydrofuran, ketones such as acetone, cyclohexanone and isophorone, esters such as ethyl acetate, ethylene glycol acetate and dibutyl maleate, alcohols such as methanol, n-hexanol and ethylene glycol, polar solvents such as dimethylformamide and dimethyl sulfoxide, water or the like. As the gas phase carrier, mixed jetting can be also effected by use of air, nitrogen, carbon dioxide, Freon, etc.

As the surfactant or dispersing agent for effecting improvement of attachment and absorption of the present agent onto animals and vegetables, improvement of performances such as dispersion, emulsification and spreading of the drug, for example, alcohol sulfates, alkylsulfonates, ligninsulfonates, polyoxyethylene glycol ether, etc. may be employed.

Further, for improving the properties of the preparation, as the auxiliary agent, for example, carboxymethyl cellulose, polyethylene glycol, gum arabic, etc. may be employed.

The above-mentioned carrier, surfactant, dispersing agent and auxiliary agent may be employed individually alone or in combination depending on the respective purposes.

The active ingredient concentration when the, compound of the present invention is formed into a preparation may be generally 1 to 50 % by weight for emulsion, generally 0.3 to 25 % by weight for powder, generally 1 to 90 % by weight for wettable agent, generally 0.5 to 5 % by weight for granule, generally 0.5 to 5 % by weight for oil agent, generally 0.1 to 5 % by weight for aerosol.

These preparations are diluted to appropriate concentrations, and can be provided for various uses depending on respective purposes by spraying on the vegetable stalk and leave, solid, water surface of paddle field, or by way of direct application.

The present invention is described below in more detail by referring to reference examples and examples, but these examples are not limitative of the scope of the present invention at all.

Reference example 1 (Synthesis of a material compound (IIa))

(1) Synthesis of dl-5-chloro-6-ethyl-4-(α-ethyl-4-hydroxybenzylamino) pyrimidine:

7 g of 4, 5-dichloro-6-ethylpyrimidine, 6 g of dl-α-ethyl-4-hydroxybenzylamine and 6 ml of triethylamine were dissolved in 100 ml of ethanol and heated under reflux for 8 hours.

After the reaction, ethanol was removed by evaporation, and ml of toluene was added, followed by extraction by use of 2 N sodium hydroxide solution. The extract was washed with water, dried over anhydrous sodium sulfate, and the solvent was removed by evaporation under reduced pressure.

The resulting crystal was recrystallized with ether-hexane to obtain 4 g of dl-5-chloro-6-ethyl-4-(α-ethyl-4-hydroxybenzylamino)pyrimidine which is a starting material (IIa) with colorless crystal.

(2) Synthesis of respective material compounds having the substituents ($R_2$, $R_3$ and $R_4$) as shown in Table 1)

Respective material compounds (IIa) having the substituents ($R_2$, $R_3$ and $R_4$) as shown in Table 1 were prepared in the same manner as in the above (1).

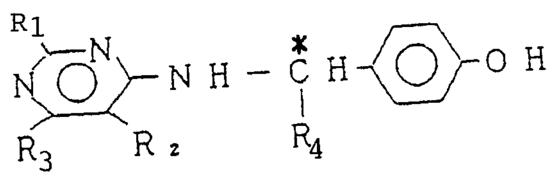

(IIa)

Example 1

(1) Synthesis of dl-5-chloro-6-ethyl-4-(α-ethyl-4-cyanomethyloxybenzylamino)pyrimidine (Compound No. 1)

0.8 g (2.7 mmol) of dl-5-chloro-6-ethyl-4-(α-ethyl-4-hydroxybenzylamino)pyrimidine which is a material compound (IIa) obtained in Reference example 1, 0.8 g (2.7 mmol) of pyrimidine, 0.3 g (4.0 mmol) of chloroacetonitrile, 1 g of potassium carbonate and 0.1 g of sodium iodide were added into 30 ml of acetone, and the mixture was heated under reflux for 8 hours. The resulting mixture was added with water and the separated oil product was extracted with toluene. The extract was washed with diluted hydrochloric acid and water. After the extract was dried with anhydrous sodium sulfate, toluene was removed under reduced pressure.

The oil product obtained was isolated by silicagel column chromatography (Wako gel C-200, eluted with toluene:ethyl acetate = 5:1) to give 0.7 g of the desired compound as colorless liquid (indicated in Table 1 as Compound No. 1).

(2) Synthesis of Compound Nos. 2 to 17

Desired compounds (Ia) (indicated in Table 1 as Compound Nos. 2 to 17) were obtained by using a material compound (IIa) of Reference example 1 and a material compound (IIIa) of Reference example 2 in the same manner as in the above (1).

Table 1

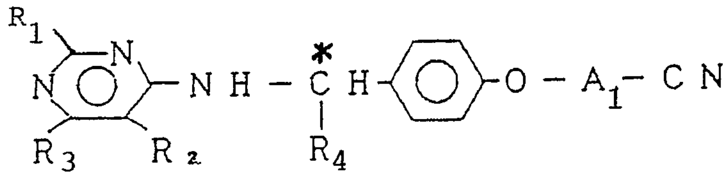 (Ia)

| Compound | $R_3$ | $R_2$ | $R_4$ | $A_1$ | Physical properties |
|---|---|---|---|---|---|
| 1 | $C_2H_5$ | Cl | $C_2H_5$ | $CH_2$ | $n_D^{26.6}$ 1.5706 |
| 2 | $CH_3$ | Cl | $C_2H_5$ | $CH_2$ | $n_D^{26.6}$ 1.5820 |
| 3 | $CH_3$ | Cl | $CH_3$ | $CH_2$ | $n_D^{17.6}$ 1.5824 |
| 4 | $CH_3$ | Cl | $i-C_3H_7$ | $CH_2$ | |
| 5 | $CH_3$ | Cl | 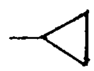 | $CH_2$ | |
| 6 | Cl | $CH_3$ | $CH_3$ | $CH_2$ | |
| 7 | Cl | $CH_3$ | $C_2H_5$ | $CH_2$ | |
| 8 | $C_2H_5$ | Cl | $C_2H_5$ | $-(CH_2)_2-$ | (*-1) |
| 9 | $C_2H_5$ | Cl | $C_2H_5$ | $-CHCH_3-$ | |
| 10 | $C_2H_5$ | Cl | $C_2H_5$ | $-(CH_2)_3-$ | $n_D^{23.0}$ 1.5601 |
| 11 | $C_2H_5$ | Cl | H | $CH_2$ | |
| 12 | $CH_3$ | F | $CH_3$ | $CH_2$ | |
| 13 | $C_2H_5$ | F | $CH_3$ | $CH_2$ | |
| 14 | $C_2H_5$ | Br | $CH_3$ | $CH_2$ | |

Table 1 (Cont'd)

| Compound | $R_3$ | $R_2$ | $R_4$ | $A_1$ | Physical properties |
|---|---|---|---|---|---|
| 15 | 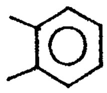 | | $CH_3$ | $CH_2$ | m.p. 52 - 55 °C |
| 16 | 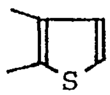 | | $CH_3$ | $CH_2$ | (*-2) |
| 17 | $C_2H_5$ | Cl | $CH_3$ | $CH_2$ | $n_D^{22.2}$ 1.5782 |

$R_1$ denotes hydrogen atom in the above table.

(*-1) $^1$H-NMR ($CDCl_3$, δ ppm)

0.93 (3H,t), 1.25 (3H,t), 1.90 (2H,m), 2.75 - 2.84 (4H,m), 4.18 (2H,t), 5.02 (1H,m), 5.58 (1H,d), 6.89 (2H,d), 7.27 (2H,d), 8.37 (1H,s)

(*-2) $^1$H-NMR ($CDCl_3$, δ ppm)

1.63 (3H,d), 4.75 (2H,s), 5.55 - 5.58 (2H,m), 6.96 (2H,d), 7.18 (1H,d), 7.27 (1H,s), 7.40 (2H,d), 8.49 (1H,s)

Example 2 (Preparation of granules)

5 parts by weight of Compound No. 1, 35 parts by weight of bentonite, 57 parts by weight of talc, 1 part by weight of Neopelex powder (trade name; manufactured by Kao Atlas) and 2 parts by weight of sodium ligninsulfonate were uniformly mixed, and then kneaded with addition of a small amount of water, followed by granulation and drying to obtain granules.

Example 3 (Preparation of wettable powder)

50 parts by weight of Compound No. 1, 48 parts by weight of kaolin and 2 parts by weight of Neopelex powder (trade name; manufactured by Kao Atlas) were uniformly mixed, followed by pulverization to obtain wettable powder.

Example 4 (Preparation of emulsion)

To 20 parts by weight of Compound No. 1 and 70 parts by weight of xylene were added 10 parts by weight of Toxanone (trade name; manufactured by Sanyo Kasei Kogyo), and the mixture was uniformly mixed and dissolved to obtain an emulsion.

Example 5 (Preparation of powder)

5 parts by weight of Compound No. 1, 50 parts by weight of talc and 45 parts by weight of kaolin were uniformly mixed to obtain powder.

Example 6

Control activity test (preventive activity) against wheat brown rust

In plastic planting pots of 6 cm in diameter, ten wheats (species: Kobushikomugi) were grown per each pot, and to young plants at the 1.5 leaf stage were sprayed the wettable powder of the respective compounds of the desired compound (I) shown in Table 1, prepared similarly as in Example 3, diluted to 50 ppm with water containing a surfactant (0.01 %) in an amount of 20 ml per each pot.

After spraying, the plants were cultivated in a glass house for 2 days, and then a wheat brown rust (Puccinia dispersa ) spore suspension (7 x $10^4$ spores/ml) was uniformly inoculated by spraying thereon.

After inoculation, the plants were grown in a glass house for one week, and the extent of the wheat brown rust lesion appeared on the first leave was examined.

Evaluation is shown by the 6 ranks of 0 to 5 judging by comparison with the extent of deseases of the non-treated group. In the standard of the comparison, one which is wholly afflicted with the disease as 0, one with lesion area of about 60 % as compared with the non-treated group as 1, about 40 % as 2, about 20 % as 3, about 10 % or less as 4, and one without lesion being rated as 5.

As control, the following compound A disclosed in Japanese Unexamined Patent Publication No. 36666/1984 and compound B disclosed in Japanese Unexamined Patent Publication No. 68362/1989 diluted to 50 ppm were used.

Compound A

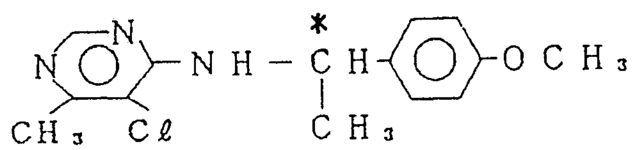

Compound B

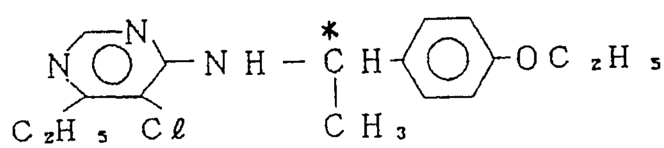

Table 2

| Compound No. | Preventive activity against wheat brown rust |
|---|---|
| 1 | 5 |
| 2 | 4 |
| 3 | 4 |
| 8 | 4 |
| 10 | 5 |
| 17 | 5 |
| Compound A | 1 |
| Compound B | 1 |
| No treatment | 0 |

Example 7

Control activity test (preventive activity) against barley powdery mildew

In plastic planting pots of 6 cm in diameter, ten barleys (species: Black barley) were grown per each pot, and to young plants at the 1.5 leaf stage was sprayed the wettable powder of the respective desired compound (I) as shown in Table 1, prepared similarly as in Example 3, diluted to 200 ppm with water containing a surfactant (0.01 %) in an amount of 20 ml per each pot.

After spraying, the plants were cultivated in a glass house for 2 days, and then conidial spores of barley powdery mildew (Erysiphe graminis ) were collected from the afflicted leaves and sprayed uniformly on the plants to effect inoculation.

After inoculation, the plants were grown in a glass house for one week, and the extent of the barley powdery mildew lesion appeared on the first leave was examined.

The results of evaluation for drug effect were judged by the same evaluation method as in Example 6 and are shown in Table 3. As a control, the same compounds A and B as in Example 6 were used provided that these compounds were diluted to 200 ppm.

Table 3

| Compound No. | Preventive activity against barley powdery mildew |
|---|---|
| 1 | 5 |
| 2 | 5 |
| 3 | 5 |
| 8 | 4 |
| 10 | 4 |
| 17 | 3 |
| Compound A | 0 |
| Compound B | 1 |
| No treatment | 0 |

Example 8

Control activity test (preventive activity) against cucumber gray mold

In plastic planting pots of 6 cm in diameter, one cucumber (species: Sagamihanjiro) was grown per each pot, and to young plants at the 1.5 leaf stage was sprayed the wettable powder of the desired compound (I) shown in table 1, prepared similarly as in Example 3, diluted to 500 ppm with water containing a surfactant (0.01 %) in an amount of 20 ml per each pot.

After spraying, the plants were cultivated in a glass house for 2 days, and then conidia suspension (5 x $10^5$ spores/ml) prepared by using cucumber gray mold (Botrytis cinerea ) lawn which was cultivated in agar culture medium, were sprayed uniformly on the plants to effect inoculation.

After inoculation, the plants were maintained for 2 days and the extent of the cucumber gray mold lesion appeared on the cotyledon was examined.

The drug effect was judged by under the same evaluation method as in Example 6 and the results are shown in Table 4.

As a control, the same compounds A and B as in Example 6 were used provided that these were diluted to 500 ppm.

Table 4

| Compound No. | Preventive activity against cucumber gray mold |
|---|---|
| 1 | 4 |
| Compound A | 0 |
| Compound B | 0 |
| No treatment | 0 |

Example 9

Control activity test (preventive activity) against rice blast

In plastic planting pots of 6 cm in diameter, ten rices (species: Nipponbare) were grown per each pot, and to young plants at the 3.0 leaf stage was sprayed the wettable powder of the desired compound (1) shown in Table 1, prepared similarly as in Example 3, diluted to 500 ppm with water containing a surfactant (0.01 %) in an amount of 20 ml per each pot.

After spraying, the plants were cultivated in a glass house for 2 days, and then conidia suspension (7 x $10^4$ spores/ml) of rice blast (Pyricularia oryzae ) were prepared from the afflicted leaves and sprayed uniformly on the plants to effect inoculation.

After inoculation, the plants were grown in a humid chamber of 28 °C for 5 days, and the extent of the rice blast lesion appeared on the third leave was examined.

The drug effect was judged under the same evaluation method as in Example 6 and the results are shown in Table 5.

As a control, the same compounds A and B as in Example 6 were used, provided that the compounds were diluted to 500 ppm.

Table 5

| Compound No. | Preventive activity against rice blast |
|---|---|
| 2 | 4 |
| 17 | 5 |
| Compound A | 0 |
| Compound B | 0 |
| No treatment | 0 |

Example 10

Control activity test (preventive activity) against cucumber downy mildew

In plastic planting pots of 6 cm in diameter, one cucumber (species: Sagamihanjiro) was grown per each pot, and to young plants at the 1.5 leaf stage was sprayed the wettable powder of the desired compound (I) as shown in Table 1, prepared similarly as in Example 2, diluted to 500 ppm with water containing a surfactant (0.01 %) in an amount of 20 ml per each pot.

After spraying, the plants were cultivated in a glass house for 2 days, and then cucumber downy mildew (Pseudoperonospora cubensis) zoosporangia were prepared from the afflicted leaves and sprayed uniformly on the plants to effect inoculation.

After inoculation, the plants were maintained under dark at 20 °C for 2 days and then grown in a glass house for five days, and the extent of the cucumber downy mildew lesion appeared on the first leave was examined.

The drug effect was judged under the same evaluation method as in Example 6 and the results are shown in Table 6.

As a control, the same compounds A and B as in Example 6 were used provided that these compounds were diluted to 500 ppm.

Table 6

| Compound No. | Preventive activity against cucumber downy mildew |
|---|---|
| 8 | 5 |
| 10 | 4 |
| Compound A | 0 |
| Compound B | 0 |
| No treatment | 0 |

Example 11

(1) Synthesis of dl-5-chloro-6-methyl-4-(α-ethyl-4-trifluoromethanesulfonyloxybenzylamino)pyrimidine (Compound No. 18)

0.4 g of dl-5-chloro-6-methyl-4-(α-ethyl-4-hydroxybenzylamino)pyrimidine was dissolved in 10 ml of pyridine. The mixture was gradually added with 0.5 g of trifluoromethanesulfonic acid anhydride under stirring with ice-cooling. The resulting mixture was stirred at a room temperature for 2 hours and added with

water, followed by extraction of the separated oily product with toluene. The extract was washed with diluted hydrochloric acid and water, dried over anhydrous sodium sulfate and then a solvent was removed by evaporation under reduced pressure.

The obtained oily product was purified by column chromatography (Wako gel C-200, eluted with toluene:ethyl acetate = 10:1) to give 0.2 g of the desired compound as colorless crystals (indicated in Table 7 as Compound No. 18).

(2) Synthesis of dl-5-chloro6-ethyl-4-(α-ethyl-4-methanesulfonyloxybenzylamino)pyrimidine (Compound No. 19)

0.8 g of dl-5-chloro-6-ethyl-4-(α-ethyl-4-hydroxybenzylamino)pyrimidine was dissolved in 10 ml of pyridine. 0.4 g of methanesulfonylchloride whcih is a starting material (IIIb) was gradually added to the mixture while stirring under ice-cooling. Then, the resulting mixture was stirred at a room temperature for 3 hours and added with water, followed by extraction of the separated oily product with toluene.

The extract was washed with diluted hydrochloric acid and water, dried over anhydrous sodium sulfate and then a solvent was removed by evaporation under reduced pressure. The resulting oily product was purified by column chromatography (Wako gel C-200, eluted with toluene:ethyl acetate = 7:1) to give 0.4 g of the desired compound as colorless crystals (indicated in Table 7 as Compound No. 19).

(3) Syntheses of Compound Nos. 20 to 39 in Table 7

The desired compound (Ib) as indicated in Table 7 (Compound Nos. 20 to 39) were prepared according to the synthesis methods as mentioned above.

Table 7

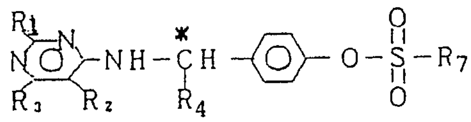 (Ib)

| Compound | 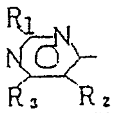 | $R_4$ | $R_7$ | Physical properties |
|---|---|---|---|---|
| 18 | 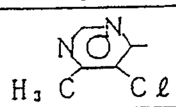 | $C_2H_5$ | $CF_3$ | m.p. 68~70 ℃ |
| 19 | 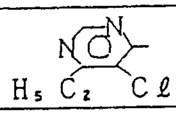 | 〃 | $CH_3$ | $n_D^{26.6}$ 1.5680 |
| 20 | 〃 | 〃 | $CF_3$ | $n_D^{22.0}$ 1.5242 |
| 21 | 〃 | $CH_3$ | 〃 | $n_D^{23.0}$ 1.5280 |
| 22 | 〃 | 〃 | $CH_3$ | m.p. 94~96 ℃ |
| 23 | 〃 | $C_2H_5$ | $C_2H_5$ | $n_D^{17.6}$ 1.5520 |
| 24 | 〃 | 〃 | $i-C_3H_7$ | $n_D^{23.2}$ 1.5571 |
| 25 | 〃 | 〃 | $n-C_4H_9$ | $n_D^{23.0}$ 1.5552 |
| 26 | 〃 | 〃 | $-(CH_2)_3Cl$ | $n_D^{26.0}$ 1.5644 |
| 27 | 〃 | $i-C_3H_7$ | $CF_3$ | |
| 28 | 〃 | 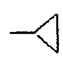 | 〃 | |
| 29 | 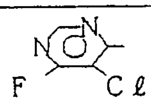 | $CH_3$ | 〃 | |
| 30 | 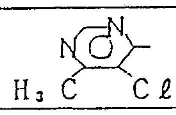 | 〃 | $CH_3$ | m.p. 127~128 ℃ |
| 31 | 〃 | $C_2H_5$ | 〃 | m.p. 119~121 ℃ |
| 32 | 〃 | 〃 | $i-C_3H_7$ | $n_D^{21.4}$ 1.5628 |
| 33 | 〃 | 〃 | $n-C_4H_9$ | m.p. 101~104 ℃ |

Table 7 (Cont'd)

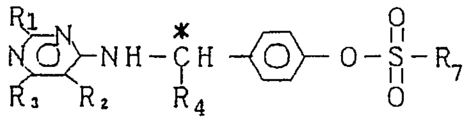 (Ib)

| Compound | 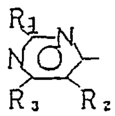 | $R_4$ | $R_7$ | Physical properties |
|---|---|---|---|---|
| 34 | 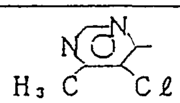 | $C_2H_5$ | 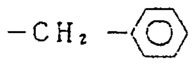 | m.p. 139~142 °C |
| 35 | 〃 | 〃 | 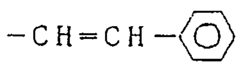 | m.p. 145~148 °C |
| 36 | 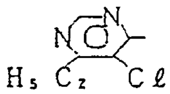 | 〃 | 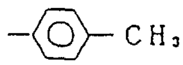 | m.p. 118~119 °C |
| 37 | 〃 | 〃 | 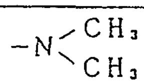 | $n_D^{23.1}$ 1.5519 |
| 38 | 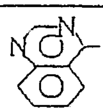 | $CH_3$ | $CH_3$ | m.p. 183~185 °C |
| 39 | 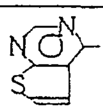 | 〃 | 〃 | m.p. 149~151 °C |

Example 12

(1) (Preparation of granules)

5 parts by weight of Compound No. 18, 35 parts by weight of bentonite, 57 parts by weight of talc, 1 part by weight of Neopelex powder (trade name; manufactured by Kao Atlas) and 2 parts by weight of sodium ligninsulfonate were uniformly mixed, and then kneaded with addition of a small amount of water, followed by granulation and drying to obtain granules.

(2) (Preparation of wettable powder)

50 parts by weight of Compound No. 18, 48 parts by weight of kaolin and 2 parts by weight of Neopelex powder (trade name; manufactured by Kao Atlas) were uniformly mixed, followed by pulverization to obtain wettable powder.

(3) (Preparation of emulsion)

To 20 parts by weight of Compound No. 18 and 70 parts by weight of xylene were added 10 parts by weight of Toxanone (trade name; manufactured by Sanyo Kasei Kogyo), and the mixture was uniformly mixed and dissolved to obtain an emulsion.

(4) (Preparation of powder)

5 parts by weight of Compound No. 18, 50 parts by weight of talc and 45 parts by weight of kaolin were uniformly mixed to obtain powder.

Example 13

(1) Control activity test (preventive activity) against wheat brown rust

In plastic planting pots of 6 cm in diameter, ten wheats (species: Kobushikomugi) were grown per each pot, and to young plants at the 1.5 leaf stage were sprayed wettable powder of Compound No. 18 in Table 7, prepared similarly as in Example 12, diluted to 50 ppm with water containing a surfactant (0.01 %) in an amount of 20 ml per each pot. After spraying, the plants were cultivated in a glass house for 2 days, and then a wheat brown rust (Puccinia recondita ) spore suspension (7 x $10^4$ spores/ml) was uniformly inoculated by spraying thereon.

After inoculation, the plants were grown in a glass house for one week, and the extent of the wheat brown rust lesion appeared on the first leaf was examined. The drug effect was judged by comparing with the extent of the no-treated group. The results are shown in Table 8.

The evaluation was indicated by 6 ranks of 5 to 0. In the standard of the comparison, one without leison being rated as 5, one with lesion area of 10 % or less as compared with the non-treated group as 4, about 20 % as 3, about 40 % as 2, about 60 % as 1, and one which is wholly afflicted with the desease as 0.

As control, the following compound A disclosed in Japanese Unexamined Patent Publication No. 36666/1984 and the compound B disclosed in Japanese Unexamined Patent Publication No. 68362/1989.

Compound A

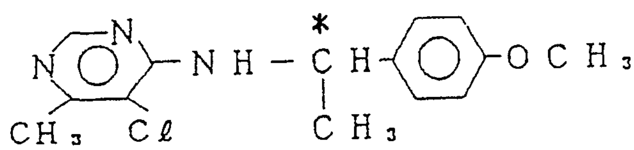

Compound B

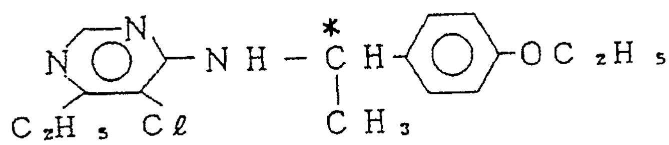

Table 8

| Compound No. | Preventive activity against wheat brown rust |
|---|---|
| 18 | 5 |
| 19 | 5 |
| 20 | 5 |
| 21 | 5 |
| 22 | 5 |
| 23 | 5 |
| 24 | 5 |
| 25 | 4 |
| 26 | 4 |
| 32 | 4 |
| 37 | 4 |
| Compound A | 1 |
| Compound B | 1 |
| No treatment | 0 |

(2) Control activity test (preventive activity) against barley powdery mildew

In plastic planting pots of 6 cm in diameter, ten barleys (species: Black barley) were grown per each pot, and to young plants at the 1.5 leaf stage was sprayed the wettable powder of the compound (Ib) in Table 7, prepared similarly as in Example 12, diluted to 100 ppm with water containing a surfactant (0.01 %) in an amount of 20 ml per each pot. After spraying, the plants were cultivated in a glass house for 2 days, and then conidial spores of barley powdery mildew (Erysiphe graminis ) were collected from the afflicted leaves and then sprayed uniformly on the plants to effect inoculation.

After inoculation, the plants were grown in a glass house for one week, and the extent of the barley powdery mildew lesion appeared on the first leaf was examined.

The results of evaluation for drug effect were judged by the same evaluation method as in the above (1) and are shown in Table 9. As a control, the same compounds as in the above (1) were used.

Table 9

| Compound No. | Preventive effect against barley powdery mildew |
|---|---|
| 18 | 5 |
| 20 | 5 |
| 21 | 5 |
| 22 | 5 |
| 23 | 5 |
| 24 | 4 |
| 26 | 4 |
| Compound A | 0 |
| Compound B | 1 |
| No treatment | 0 |

(3) Control activity test (preventive activity) against cucumber downy mildew

In plastic planting pots of 6 cm in diameter, one cucumber (species: Sagamihanjiro) was grown per each pot, and to young plants at the 1.5 leaf stage was sprayed the wettable powder of the desired compound (Ib) as shown in Table 7, prepared similarly as in Example 12, diluted to 500 ppm with water containing a surfactant (0.01 %) in an amount of ml per each pot.

After spraying, the plants were cultivated in a glass house for 2 days, and then cucumber downy mildew (Pseudoperonospora cubensis ) zoosporangia were prepared from the afflicted leaves and sprayed uniformly on the plants to effect inoculation.

After inoculation, the plants were maintained under dark at 20 °C for 2 days and then grown in a glass house for five days, and the extent of the cucumber downy mildew lesion appeared on the first leave was examined.

The drug effect was judged under the same evaluation method as in the above (1) and the results are shown in Table 10.

As a control, the same compounds A and B as in the above (1) were used.

Table 10

| Compound No. | Preventive activity against cucumber downy mildew |
|---|---|
| 21 | 5 |
| 24 | 5 |
| 25 | 5 |
| 37 | 5 |
| 39 | 5 |
| Compound A | 0 |
| Compound B | 0 |
| No treatment | 0 |

(4) Control activity test (preventive activity) against rice blast

In plastic planting pots of 6 cm in diameter, ten rices (species: Nipponbare) were grown per each pot, and to young plants at the 3.0 leaf stage was sprayed the wettable powder of the desired compound (Ib) shown in Table 7, prepared similarly as in Example 12, diluted to 500 ppm with water containing a surfactant (0.01 %) in an amount of 20 ml per each pot.

After spraying, the plants were cultivated in a glass house for 2 days, and then conidia suspension (7 x $10^4$ spores/ml) of rice blast (Pyricularia oryzae ) were sprayed uniformly on the plants to effect inoculation.

After inoculation, the plants were grown in a humid chamber of 28 °C for 5 days, and the extent of the rice blast lesion appeared on the third leaf was examined.

The drug effect was judged under the same evaluation method as in the above (1) and the results are shown in Table 11. As a control, the same compounds A and B as in the above (1) were used.

Table 11

| Compound No. | Preventive activity against rice blast |
|---|---|
| 24 | 5 |
| 26 | 5 |
| 37 | 5 |
| Compound A | 0 |
| Compound B | 0 |
| No treatment | 0 |

Reference example 2

Synthesis of a material compound (IIIc)

(1) Synthesis of 3,4-(difluoromethylenedioxy)benzylamine

0.5 g (13 mmol) of lithium aluminum hydride was suspended in 10 ml anhydrous ethyl ether. To the suspension, a solution of 1 g (5.5 mmol) of 3,4-(difluoromethylenedioxy)benzonitrile dissolved in anhydrous ethyl ether was gradually dropwise added and the resulting liquid was heated under reflux for 2 hours.

After the reaction, excess amount of lithium aluminum hydride was decomposed by ethanol and subsequently water, followed by extraction with ethyl ether.

The resulting extract was dried over anhydrous sodium sulfate and the solvent was removed be evaporation under reduced pressure, followed by purification by column chlomatography to obtain 0.5 g of 3,4-(difluoromethylenedioxy)benzylamine as colorless oily products.
C1 - Mass (m/e) 188 ($M^+$ +1), 171 ($M^+$ -16)

(2) Synthesis of α-ethyl-3,4-(difluoromethylenedioxy)benzylamine

1.3 g (5.5 mmol) of 3,4-(difluoromethylenedioxy)propiophenone oxime and 1 g of Raney nickel were suspended in 50 ml of ethanol and the suspension was saturated with ammonia gas. The resulting mixture was stirred in an autoclave under hydrogen pressure of 30 kg/cm$^2$ at 100 °C for 6 hours.

After the reaction, insolubles were removed by filtration and solvent was removed by evaporation, followed by purification by column chromatography to obtain 1 g of α-ethyl-3,4-(difluoromethylenedioxy)-benzylamine as colorless oily products.
C1 - Mass (m/e) 216 ($M^+$ +1), 199 ($M^+$ -16) 186 ($M^+$ -29)

(3) Synthesis of α-(2,2,3-trifluorobenzo-1,4-dioxane-6-yl)ethylamine

6.7 g of 6-( α-hydroxyiminoethyl)-2,2,3-trifluorobenzo-1,4-dioxane and 4 g of Raney nickel were suspended in 70 ml of ethanol, and the suspension was saturated with ammonia gas. The resulting mixture was stirred in an autoclave under hydrogen pressure of 30 kg/m$^2$ at 100 °C for 6 hours.

After the reaction, insolubles were removed by filtration and solvent was removed by evaporation, followed by purification by column chromatography to obtain 5.5 g of α-2,2,3-trifluorobenzo-1,4-dioxane-6-yl)ethylamine as colorless oily products.
C1 - Mass (m/e) 233 ($M^+$ +1), 217 ($M^+$ -15)

(4) Synthesis of 3,4-difluoromethylenedioxy)propiophenone oxime

1.5 g (7 mmol) of 3,4-difluoromethylenedioxy)propiophenone, 1.5 g (22 mmol) of hydroxylamine hydrochloride and 1.5 g (36 mmol) of sodium hydroxide were dissolved in a mixture of ethanol and water (ethanol 15 ml - water 5 ml) and stirred at 70 °C for 3 hours.

After the reaction, solvent was removed by evaporation, followed by extraction with acetate. The resulting extract was washed with water, dried over anhydrous sodium sulfate and the solvent was removed by evaporation under reduced pressure. The separated crystal was washed with hexane to obtain 1.3 g of 3,4-(difluoromethylenedioxy)propiophenone oxime as colorless crystals.
C1 - Mass (m/e) 230 ($M^+$ +1), 212 ($M^+$ -17) 184 ($M^+$ -55)

(5) Synthesis of 6-(α-hydroxyiminoethyl)2,2,3-trifluorobenzo-1,4-dioxane

7 g (30 mmol) of 6-acetyl-2,2,3-trifluorobenzo-1,4-dioxane, 3 g (43 mmol) of hydroxylamine hydrochloride and 1.7 g (43 mmol) of sodium hydroxide were dissolved in a mixture of ethanol and water (ethanol 50 ml - water 10 ml), and the mixture was stirred at 70 °C for 3 hours.

After the reaction, the solvent was removed by evaporation, followed by extraction with ethyl acetate. The resulting extract was washed with water, dried over anhydrous sodium sulfate and the solvent was removed by evaporation under reduced pressure. The separated crystal was washed with hexane to obtain 6.5 g of 6-(α-hydroxyimino-ethyl)2,2,3-trifluorobenzo-1,4-dioxane as colorless crystals.
C1 - Mass (m/e) 248 ($M^+$ +1), 230 ($M^+$ -17)

(6) Synthesis of 3,4-(difluoromethylenedioxy)propiophenone

To an ether solution of ethylmagnesiumbromide [EtMgBr (12 mmol)], a solution of 1.2 g of 3,4-(difluoromethylenedioxy)benzonitrile dissolved in 8 ml of anhydrous ether was gradually dropwise added. After heating for 2 hours under reflux, the resulting solution was poured into ice-water, followed by extraction with ethyl acetate.

The resulting extract was washed with water and dried over anhydrous sodium sulfate, and the solvent was removed under reduced pressure. The separated crystal was washed with hexane to obtain 1.4 g of 3,4-(difluoromethylenedioxy)propiophenone as colorless crystals.
C1 - Mass (m/e) 215 ($M^+$ +1), 185 ($M^+$ -29)

(7) Synthesis of 6-acetyl-2,2,3-trifluorobenzo-1,4-dioxane

10 g (66 mmol) of 3,4-dihydroxyacetophenone and 4.4 g (78 mmol) of potassium hydroxide powder were suspended in 30 ml of sulforane. The suspension obtained was heated to 90 °C under nitrogen atmosphere. To the resulting mixture, trifluorochloroethylene heated to 100 to 110 °C was brown until 3,4-dihydroxyacetophenone as a starting material was consumed.

After cooling, the resulting solution was extracted with toluene. The extract was washed with water and dried over anhydrous sodium sulfate, and the solvent was removed by evaporation. The obtained oily product was purified by column chromatography (Wake gel C-200, eluted with toluene:ethyl acetate = 10:1) to obtain 7 g of 6-acetyl- 2,2,3-trifluorobenzo-1,4-dioxane as colorless oily products.
C1 - Mass (m/e) 233 ($M^+$ +1), 218 ($M^+$ -15)

Example 14

(1) Synthesis of 5-chloro-6-ethyl-4-[α-(2,2,3-trifluorobenzo-1,4-dioxane-6-yl) ethylamino] pyrimidine

0.7 g (4.0 mmol) of 4,5-dichloro-6-ethylpyrimidine which is a material compound (IIb), 0.8 g (3.4 mmol) of 1-(2,2,3-trifluorobenzo-1,4-dioxane-6-yl)ethylamine which is a material compound (IIIc) and a catalytic amount of 4-(N,N-dimethylamino)pyridine were suspended in 5 ml of triethylamine and the suspension was heated under reflux for 5 hours.

After the reaction, extraction with toluene and washing with water were conducted. After drying with anhydrous sodium sulfate, toluene was removed by evaporation under reduced pressure.

The resulting oily product was purified by silicagel column chromatography (Wako gel C-200, eluted with toluene:ethyl acetate = 5:1) to give 0.5 g of the desired compound as colorless oily product (indicated in Table 12 as Compound No. 41).

(2) Synthesis of 4-[α-(2,2,3-trifluorobenzo-1,4-dioxane-6-yl)ethylamino]thieno[2,3-d]pyrimidine

0.65 g (3.8 mmol) of 4-chlorothieno[2,3-d]pyrimidine, 0.8 g (3.4 mmol) of 1-(2,2,3-trifluorobenzo-1,4-dioxane-6-yl)ethylamine, 1 ml of triethylamine and a catalytic amount of 4-(N,N-dimethyl-amino)pyridine were dissolved in 5 ml of dimethylformamide and the solution was stirred with heating at 60 to 80 °C for 8 hours.

After the reaction, water was added to the mixture and extraction with toluene and washing with water were conducted. After drying over anhydrous sodium sulfate, toluene was removed under reduced pressure.

The resulting oily product was purified by silicagel column chromatography (Wako gel C-200, eluted with toluene:ethyl acetate = 3:1) and crystallized with n-hexane to give 0.8 g of the desired compound as

colorless crystals (indicated in Table 12 as Compound No. 42).

(3) Synthesis of 5-chloro-6-mehtyl-4-[α-ethyl-3,4-(difluoromethylenedioxy)benzylamino]pyrimidine

0.5 g (3 mmol) of 4,5-dichloro-6-methylpyrimidine which is a material compound (IIb), 0.5 g (2.3 mmol) of α-ethyl-3,4-(difluoromethylenedioxy)benzylamine which is a material compound (IIIc) and 5 ml of trimethylamine were dissolved in 5 ml of ethanol and the solution was heated under reflux for 5 hours.

After the reaction, the mixture was poured into water, above subjected to extraction with toluene and washed with water. After drying over anhydrous sodium sulfate, toluene was removed by evaporation under reduced pressure.

The resulting oily product was purified by silicagel column chromatography (Wako gel C-200, eluted with toluene:ethyl acetate = 4:1) to give 0.5 g of the desired compound as a colorless oily products (indicated in Table 12 as Compound No. 48).

(4) Synthesis of 5-chloro-6-ethyl-4-[3,4-(difluoromethylenedioxy)benzylamino]pyrimidine

0.6 g (3.3 mmol) of 4,5-dichloro-6-ethylpyrimidine which is a material compound (IIb), 0.5 g (2.8 mmol) of 3,4-(difluoromethylenedioxy)benzylamine which is a material compound (IIIc) and a catalytic amount of 4-(N,N-dimethylamino) pyrimidine were suspended in 5 ml of triethylamine and the suspension was heated under reflux for 5 hours.

After the reaction, extraction with toluene and washing with water were conducted after drying with anhydrous sodium sulfate, the toluene was removed by evaporation under reduced pressure.

The resulting oily product was purified by silicagel column chromatography (Wako gel C-200, eluted with toluene:ethyl acetate = 4:1) to give 0.5 g of the desired compound as colorless oily products (indicated in Table 12 as Compound No. 58).

(5) Syntheses of Compound Nos. 40, 43 and 49 in Table 12

The desired compounds (Ic) as shown in Table 12 (indicated as Compound Nos. 40, 43 and 49 in Table 12) in the same manner as in any synthesis method of the above (1) to (4).

Table 12

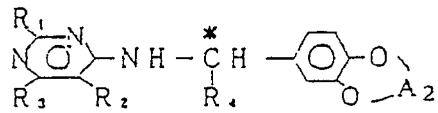 (Ic)

| Compound | $R_1$ | $R_2$ | $R_3$ | $R_4$ | 4-position — $A_2$ — 3-position | Physical properties |
|---|---|---|---|---|---|---|
| 40 | H | $Cl$ | $CH_3$ | $CH_3$ | $-CF_2-CHF-$ | $n_D^{25.2}$ 1.5506 |
| 41 | 〃 | 〃 | $C_2H_5$ | 〃 | 〃 | $n_D^{25.3}$ 1.5438 |
| 42 | 〃 | 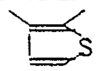 | | 〃 | 〃 | m.p. 143～146 ℃ |
| 43 | 〃 | 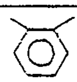 | | 〃 | 〃 | m.p. 135～138 ℃ |
| 44 | 〃 | $Cl$ | $CH_3$ | 〃 | $-CF_2-$ | |
| 45 | 〃 | 〃 | $C_2H_5$ | 〃 | 〃 | |
| 46 | 〃 | 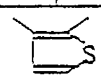 | | 〃 | 〃 | |
| 47 | 〃 | 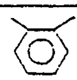 | | 〃 | 〃 | |
| 48 | 〃 | $Cl$ | $CH_3$ | $C_2H_5$ | 〃 | $n_D^{24.6}$ 1.5448 |
| 49 | 〃 | 〃 | $C_2H_5$ | 〃 | 〃 | $n_D^{24.4}$ 1.5404 |
| 50 | $CH_3$ | 〃 | $CH_3$ | $CH_3$ | 〃 | |
| 51 | $Cl$ | 〃 | 〃 | 〃 | 〃 | |
| 52 | F | 〃 | 〃 | 〃 | 〃 | |
| 53 | H | 〃 | 〃 | $i-C_3H_7$ | 〃 | |
| 54 | 〃 | 〃 | 〃 | —◁ | 〃 | |
| 55 | 〃 | 〃 | 〃 | $C_2H_5$ | $-CF_2-CHF-$ | |
| 56 | 〃 | 〃 | 〃 | $i-C_3H_7$ | 〃 | |
| 57 | 〃 | 〃 | 〃 | —◁ | 〃 | |
| 58 | 〃 | 〃 | $C_2H_5$ | H | $-CF_2-$ | $n_D^{28.8}$ 1.5549 |

Example 15

(1) Preparation of granules

5 parts by weight of Compound No. 40, 35 parts by weight of bentonite, 57 parts by weight of talc, 1 part by weight of Neopelex powder (trade name; manufactured by Kao Atlas) and 2 parts by weight of sodium ligninsulfonate were uniformly mixed and then kneaded with addition of a small amount of water, followed by granulation and drying to obtain granules.

(2) Preparation of wettable powder

10 parts by weight of Compound No. 41, 70 parts by weight of kaolin, 18 parts by weight of white carbon, 1.5 parts by weight of Neopelex powder (trade name; manufactured by Kao Atlas), 0.5 part by weight of Dennol (trade name: manufactured by Kao Atlas) were uniformly mixed, followed by pulverization to obtain wettable powder.

(3) Preparation of emulsion

To 20 parts by weight of Compound No. 40 and 70 parts by weight of xylene were added 10 parts by weight of Toxanone (trade name; manufactured by Sanyo Kasei Kogyo), and the mixture was uniformly mixed and dissolved to obtain an emulsion.

(4) Preparation of powder

5 parts by weight of Compound No. 40, 50 parts by weight of talc and 45 parts by weight of kaolin were uniformly mixed to obtain powder.

Example 16

(1) Control activity test (preventive activity) against wheat brown rust

In plastic planting pots of 6 cm in diameter, ten wheats (species: Kobushikomugi) were grown per each pot, and to young plants at the 1.5 leaf stage were sprayed the wettable powder of the respective compounds of the desired compound (Ic) shown in Table 12, prepared similarly as in Example 15, diluted to 100 ppm with water containing a surfactant (0.01 %) in an amount of 20 ml per each pot.

After spraying, the plants were cultivated in a glass house for 2 days and then a wheat brown rust (Puccinia recondita ) spore suspension (7 x $10^4$ spores/ml) was uniformly inoculated by spraying thereon.

After inoculation, the plants were grown in a glass house for one week, and the extent of the wheat brown rust lesion appeared on the first leave was examined.

Evaluation is shown by the 6 ranks of 5 to 0 judging by comparison with the extent of deseases of the non-treated group. In the standard of the comparison, one which is wholly afflicted with the disease as 0, one with lesion area of about 60 % as compared with the non-treated group as 1, about 40 % as 2, about 20 % as 3, 10 % or less as 4, and one without lesion being rated as 5. The results are shown in Table 13.

As control, the following compound A disclosed in Japanese Unexamined Patent Publication No. 36666/1984 and compound B disclosed in Japanese Unexamined Patent Publication No. 68362/1989.

Compound A

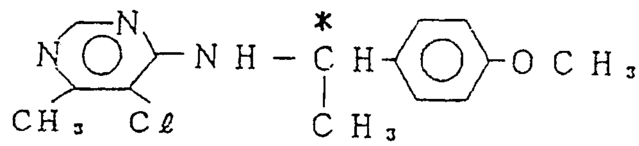

Compound B

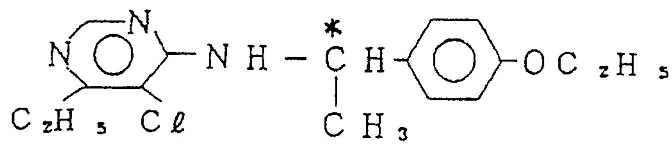

Table 13

| Compound No. | Preventive activity against wheat brown rust |
|---|---|
| 40 | 5 |
| 41 | 5 |
| 42 | 4 |
| 48 | 5 |
| 49 | 5 |
| 58 | 4 |
| Compound A | 1 |
| Compound B | 1 |
| No treatment | 0 |

(2) Control activity test (preventive activity) against barley powdery mildew

In plastic planting pots of 6 cm in diameter, ten barleys (species: Black barley) were grown per each pot, and to young plants at the 1.5 leaf stage was sprayed the wettable powder of the respective compounds of the desired compound (Ic) as shown in Table 12, prepared similarly as in Example 15, diluted to 50 ppm with water containing a surfactant (0.01 %) in an amount of 20 ml per each pot.

After spraying, the plants were cultivated in a glass house for 2 days, and then conidial spores of barley powdery mildew (Erysiphe graminis ) were collected from the afflicted leaves, which were sprayed uniformly on the plants to effect inoculation.

After inoculation, the plants were grown in a glass house for one week, and the extent of the barley powdery mildew lesion appeared on the first leave was examined.

The results of evaluation for drug effect were judged by the same evaluation method as in the above (1) and are shown in Table 14. As a control, the same compounds A and B as in the above (1) were used.

Table 14

| Compound No. | Preventive effect against barley powdery mildew |
|---|---|
| 40 | 5 |
| 41 | 5 |
| 42 | 4 |
| 48 | 4 |
| 49 | 5 |
| Compound A | 1 |
| Compound B | 1 |
| No treatment | 0 |

(3) Control activity test (preventive activity) against against cucumber downy mildew

In plastic planting pots of 6 cm in diameter, one cucumber (species: Sagamihanjiro) was grown per each pot, and to young plants at the 1.5 leaf stage was sprayed the wettable powder of the desired compound (Ic) as shown in Table 12, prepared similarly as in Example 15, diluted to 500 ppm with water containing a surfactant (0.01 %) in an amount of 20 ml per each pot.

After spraying, the plants were cultivated in a glass house for 2 days, and then cucumber downy mildew zoosporangia (Pseudoperonospora cubensis ) were prepared from the afflicted leaves and sprayed uniformly on the plants to effect inoculation.

After inoculation, the plants were maintained under dark at 20 °C for 2 days and grown in a glass house for five days, and the extent of the cucumber downy mildew lesion appeared on the first leaf was examined.

The drug effect was judged under the same evaluation method as in the above (1) and the results are shown in Table 15.

As a control, the same compounds A and B as in the above (1) were used.

Table 15

| Compound No. | Preventive activity against cucumber downy mildew |
|---|---|
| 42 | 5 |
| 43 | 5 |
| Compound A | 0 |
| Compound B | 0 |
| No treatment | 0 |

(4) Control activity test (preventive activity) against rice blast

In plastic planting pots of 6 cm in diameter, ten rices (species: Nipponbare) were grown per each pot, and to young plants at the 3.0 leaf stage was sprayed the wettable powder of the desired compound (1) shown in Table 12, prepared similarly as in Example 15, diluted to 500 ppm with water containing a surfactant (0.01 %) in an amount of ml per each pot.

After spraying, the plants were cultivated in a glass house for 2 days, and then conidia suspension (7 x $10^4$ spores/ml) of rice blast (Pyricularia oryzae ) were sprayed uniformly on the plants to effect inoculation.

After inoculation, the plants were grown in a humid chamber of 28 °C for 5 days, and the extent of the rice blast lesion appeared on the third leaf was examined.

The drug effect was judged under the same evaluation method as in the above (1) and the results are shown in Table 16. As a control, the same compounds A and B as in the above (1) were used.

Table 16

| Compound No. | Preventive activity against rice blast |
|---|---|
| 48 | 5 |
| 49 | 5 |
| 58 | 5 |
| Compound A | 0 |
| Compound B | 0 |
| No treatment | 0 |

(5) Activity test against brown planthoppers

The wettable powder of the desired compound (Ic) as shown in Table 12, prepared according to Example 15, was diluted with water containing a surfactant (0.01 %) to 1,000 ppm to prepare a chemical solution. In each chemical solution, rice young seedlings were dipped for 30 seconds. After air drying, it was placed in a glass cylinder.

Ten 3rd brown planthoppers were freed in the cylinder, and the cup was closed with a porous stopper and left to stand in a thermostatic chamber of 25 °C. Four days later, the numbers of live and dead insects were counted to determine the % mortality. The results are shown in Table 17.

The evaluation of activity is indicated by 4 ranks, in which mortality of 100 % are shown as A, those with 99 to 80 % as B, those with 79 to 60 % as C and those with not more than 59 % as D.

Table 17

| Compound No. | Activity against brown planthopper |
|---|---|
| 40 | B |
| 41 | B |
| 48 | B |
| 49 | A |

(6) Activity test against female adult of two spotted spider mite

The desired compounds (Ic) shown in Table 12 were formulated into wettable powder similarly as described in Example 15, and each formulation was diluted with water containing a surfactant (0.01 %) to 300 ppm to prepare a chemical solution. In each chemical solution, a kidney bean leaf (20 mm in diameter), on which female adult of two spotted spider mites parasitized, was dipped for 15 seconds.

These were placed in a thermostatic chamber of 25 °C. Three days later, the numbers of live and dead mites were counted to determine the % mortality. The results are shown in Table 18. The results are shown in Table 18 according to the 4 grade evaluation method indicated in the above (5).

Table 18

| Compound No. | Activity against two spotted spider mite |
|---|---|
| 40 | A |
| 41 | A |
| 48 | A |
| 49 | A |

Reference example 3 [Synthesis of a material compound (IIId)]

(1) Synthesis of dl-α-methyl-4-methylthiobenzylamine

7.6 g (0.2 mmol) of lithium aluminum hydride was suspended in 300 ml of ethyl ether. After stirring at a room temperature, ether solution of 18 g (0.1 mmol) of 4-methylthioacetophenonoxime was gradually dropwise added with stirring at a room temperature to the suspension. After the dropwise addition was completed, the mixture was heated under reflux for 5 hours.

After the reaction, excess amount of the lithium aluminum hydride was decomposed with ethanol and subsequently water.

The insolubles were removed by filtration off with Celite and the filtrate was extracted with 2N-hydrochloric acid. The extract was made alkaline with 2N-sodium hydroxide and extracted over ethyl acetate. After the extract was dried with anhydrous sodium sulfate, the solvent was removed by evaporation.

The resulting oily product was purified by silicagel column chromatography (Wako gel C-200, eluted with ethanol) to give 5 g of dl-α-methyl-4-methylthiobenzylamine which is the material compound (IIId).

Example 17

(1) Synthesis of dl-5-chloro-6-methyl-4-(α-methyl-4-methylthiobenzylamino)pyrimidine (Compound No. 59)

A mixture of 1 g (6 mmol) of 4,5-dichloro-6-methylpyrimidine which is a material compound (IIc), 0.6 g (3.6 mmol) of dl-α-methyl-4-methylthiobenzylamine which is a material compound (IIId), 5 ml of triethylamine and a catalytic amount of 4-(N,N-dimethylaminopyridine) was heated under reflux for 8 hours.

After the reaction, extraction with toluene and washing with water were conducted, followed by drying over anhydrous sodium sulfate. The solvent was removed under reduced pressure and the resulting oily product was purified by silicagel column chromatography (Wako gel C-200, eluted with toluene:ethyl acetate = 4:1) and crystallized with hexane to give 0.55 g of the desired compound as colorless crystals (indicated in Table 19 as Compound No. 59).

(2) Synthesis of dl-5-chloro-6-ethyl-4-(α-methyl-4-methylthiobenzylamino)pyrimidine (Compound No. 62)

2 g (3.3 mmol) of 4,5-dichloro-6-ethylpyrimidine which is a material compound (IIc), 1.9 g (11 mmol) of dl-α-methyl-4-methylthiobenzylamine which is a material compound (IIId), 2 ml of triethylamine and a catalytic amount of 4-(N,N-dimethylamino)pyridine were dissolved in 10 ml of N,N-dimethylformamide and the solution was heated under reflux for 8 hours.

After the reaction, extraction with toluene and washing with water were conducted, followed by drying over anhydrous sodium sulfate. The solvent was removed under reduced pressure and the resulting oily product was purified by silicagel column chromatography (Wako gel C-200, eluted with toluene:ethyl acetate = 4:1) . The resultant was crystallized with hexane to give 1.8 g of the desired compound as colorless

crystals (indicated in Table 19 as Compound No. 62).

(3) Synthesis of dl-5-chloro-6-ethyl-4-(α-methyl-4-methanesulfinylbenzylamino)pyrimidine (Compound No. 63)

0.3 g (1 mmol) of dl-5-chloro-6-ethyl-4-(α-methyl-4-methylthiobenzylamino)pyrimidine (Compound No. 62) was dissolved in 10 ml of methylene chloride. After stirring at a room temperature, 0.15 g (1 mmol) of m-chloro perbenzoic acid was added. After stirring for 5 hours, the reaction solution was extracted with chloroform. The extract was washed with 2N-sodium hydroxide and water and dried over anhydrous sodium sulfate. The solvent was removed under reduced pressure and the resulting oily product was purified by silicagel column chromatography (Wako gel C-200, eluted with ethyl acetate:ethanol = 4:1) to give 80 mg of the desired compound as colorless crystals (indicated in Table 19 as Compound No. 63).

(4) Synthesis of dl-5-chloro-6-ethyl-4-(α-methyl-4-methanesulfonylbenzylamino)pyrimidine (Compound No. 64)

1.4 g (4.6 mmol) of dl-5-chloro-6-ethyl-4-(α-methyl-4-methylthiobenzylamino)pyrimidine (Compound No. 62) was dissolved in 30 ml of methylene chloride. To the mixture, 1.7 g (6.9 mmol) of m-chloro perbenzoic acid was added under stirring at a room temperature. After stirring for 5 hours, the reaction solution was extracted with chloroform. The extract was washed with 2N-sodium hydroxide solution and water, followed by drying over anhydrous sodium sulfate. The solvent was removed under reduced pressure and the resulting oily product was purified by silicagel column chromatography (Wako gel C-200, eluted with toluene:ethyl acetate = 3:2) to give 0.32 g of the desired compound as colorless crystals (indicated in Table 19 as Compound No. 64).

(5) Synthesis of Compound Nos. 60, 61, 65 and 66 in Table 19

Desired compounds (Id) (indicated in Table 19 as Compound Nos. 60, 61, 65 and 66) were obtained according to any synthetic method in the above (1) to (4).

Table 19

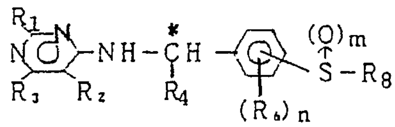 (Id)

| Compound | 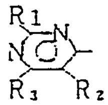 | $R_8$ | $R_4$ | $(R_6)n$ | $-\overset{(O)_m}{\overset{\uparrow}{S}}-R_8$ substitution position | m | Physical properties |
|---|---|---|---|---|---|---|---|
| 59 | 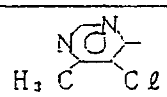 | $CH_3$ | $CH_3$ | H | 4 | 0 | m.p. 92~ 93 ℃ |
| 60 | 〃 | $C_2H_5$ | 〃 | 〃 | 〃 | 〃 | $n_D^{25.2}$ 1.6084 |
| 61 | 〃 | $-CH_2CH_2OC_2H_5$ | 〃 | 〃 | 〃 | 〃 | $n_D^{25.4}$ 1.5910 |
| 62 | 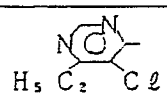 | $CH_3$ | 〃 | 〃 | 〃 | 〃 | m.p. 59~ 61 ℃ |
| 63 | 〃 | 〃 | 〃 | 〃 | 〃 | 1 | m.p. 118~122 ℃ |
| 64 | 〃 | 〃 | 〃 | 〃 | 〃 | 2 | m.p. 121~123 ℃ |
| 65 | 〃 | $C_2H_5$ | 〃 | 〃 | 〃 | 0 | $n_D^{25.1}$ 1.5964 |
| 66 | 〃 | $-CH_2CH_2OC_2H_5$ | 〃 | 〃 | 〃 | 〃 | $n_D^{25.4}$ 1.5838 |
| 67 | 〃 | $n-C_3H_7$ | 〃 | 〃 | 〃 | 〃 | |
| 68 | 〃 | $i-C_3H_7$ | 〃 | 〃 | 〃 | 〃 | |
| 69 | 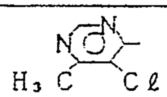 | $CH_3$ | $C_2H_5$ | 〃 | 〃 | 〃 | |
| 70 | 〃 | 〃 | $i-C_3H_7$ | 〃 | 〃 | 〃 | |
| 71 | 〃 | 〃 | 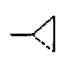 | 〃 | 〃 | 〃 | |
| 72 | 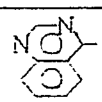 | 〃 | $CH_3$ | 〃 | 〃 | 〃 | |
| 73 | 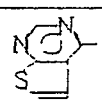 | 〃 | 〃 | 〃 | 〃 | 〃 | |

Example 18

(1) Preparation of granules

5 parts by weight of Compound No. 59, 35 parts by weight of bentonite, 57 parts by weight of talc, 1

part by weight of Neopelex powder (trade name; manufactured by Kao Atlas) and 2 parts by weight of sodium ligninsulfonate were uniformly mixed, and then the mixture was kneaded with addition of a small amount of water, followed by granulation and drying to obtain granules.

(2) Preparation of wettable powder

50 parts by weight of Compound No. 59, 48 parts by weight of kaolin and 2 parts by weight of Neopelex powder (trade name; manufactured by Kao Atlas) were uniformly mixed, followed by pulverization to obtain wettable powder.

(3) Preparation of emulsion

To 20 parts by weight of Compound No. 59 and 70 parts by weight of xylene were added 10 parts by weight of Toxanone (trade name; manufactured by Sanyo Kasei Kogyo), and the mixture was uniformly mixed and dissolved to obtain an emulsion.

(4) Preparation of powder

5 parts by weight of Compound No. 59, 50 parts by weight of talc and 45 parts by weight of kaolin were uniformly mixed to obtain powder.

Example 19

(1) Control activity test (preventive activity) against wheat brown rust

In plastic planting pots of 6 cm in diameter, ten wheats (species: Kobushikomugi) were grown per each pot, and to young plants at the 1.5 leaf stage were sprayed the wettable powder of the respective compounds of the desired compound (Id) shown in Table 19, prepared similarly as in Example 18, diluted to 50 ppm with water containing a surfactant (0.01 96) in an amount of 20 ml per each pot.

After spraying, the plants were cultivated in a glass house for 2 days, and then a wheat brown rust (Puccinia recondita ) spore suspension (7 x $10^4$ spores/ml) was uniformly inoculated by spraying thereon.

After inoculation, the plants were grown in a glass house for one week, and the extent of the wheat brown rust lesion appeared on the first leaf was examined.

Evaluation is shown by the 6 ranks of 5 to 0 judging by comparison with the extent of deseases of the non-treated group. In the standard of the comparison, one which is wholly afflicted with the disease as 0, one with lesion area of about 60 % as compared with the non-treated group as 1, about 40 % as 2, about 20 % as 3, 10 % or less as 4, and one without lesion being rated as 5. The results are shown in Table 20.

As control, the following compound A disclosed in Japanese Unexamined Patent Publication No. 36666/1984 and compound B disclosed in Japanese Unexamined Patent Publication No. 68362/1989.

Compound A

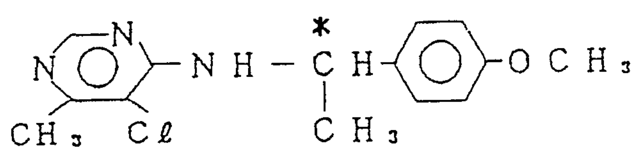

Compound B

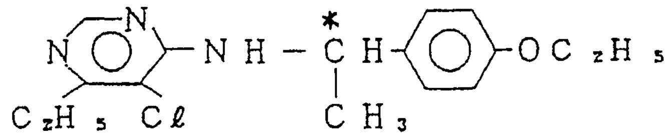

Table 20

| Compound No. | Preventive activity against wheat brown rust |
|---|---|
| 61 | 4 |
| 62 | 5 |
| 63 | 5 |
| 64 | 5 |
| 66 | 5 |
| Compound A | 1 |
| Compound B | 1 |
| No treatment | 0 |

(2) Control activity test (preventive activity) against barley powdery mildew

In plastic planting pots of 6 cm in diameter, ten barleys (species: Black barley) were grown per each pot, and to young plants at the 1.5 leaf stage was sprayed the wettable powder of the respective compounds of the desired compound (Id) as shown in Table 19, prepared similarly as in Example 18, diluted to 50 ppm with water containing a surfactant (0.01 %) in an amount of 20 ml per each pot.

After spraying, the plants were cultivated in a glass house for 2 days, and then conidial spores of barley powdery mildew (Erysiphe graminis ) were collected from the afflicted leaves and sprayed uniformly on the plants to effect inoculation.

After inoculation, the plants were grown in a glass house for one week, and the extent of the barley powdery mildew lesion appeared on the first leaf was examined.

The results of evaluation for drug effect were judged by the same evaluation method as in the above (1) and are shown in Table 21. As a control, the same compounds A and B as in the above (1) were used.

Table 21

| Compound No. | Preventive activity against barley powdery mildew |
|---|---|
| 63 | 4 |
| 64 | 4 |
| 65 | 4 |
| Compound A | 1 |
| Compound B | 1 |
| No treatment | 0 |

(3) Control activity test (preventive activity) against cucumber downy mildew

In plastic planting pots of 6 cm in diameter, one cucumber (species: Sagamihanjiro) was grown per each pot, and to young plants at the 1.5 leaf stage was sprayed the wettable powder of the desired compound (Id) as shown in Table 19, prepared similarly as in Example 18, diluted to 500 ppm with water containing a surfactant (0.01 %) in an amount of ml per each pot.

After spraying, the plants were cultivated in a glass house for 2 days, and then cucumber downy mildew (Pseudoperonospora cubensis ) zoosporangia were prepared from the afflicted leaves, which were sprayed uniformly on the plants to effect inoculation.

After inoculation, after maintained under dark at 20 °C for 2 days, the plants were grown in a glass house for five days, and the extent of the cucumber downy mildew lesion appeared on the first leaf was examined.

The drug effect was judged under the same evaluation method as in the above (1) and the results are shown in Table 22.

As a control, the same compounds A and B as in the above (1) were used.

Table 22

| Compound No. | Preventive activity against cucumber downy mildew |
|---|---|
| 62 | 4 |
| 63 | 5 |
| 64 | 4 |
| Compound A | 0 |
| Compound B | 0 |
| No treatment | 0 |

Reference example 4 [Synthesis of a material compound (IIId)]

(1) Synthesis of dl-α-methyl-4-difluoromethylythiobenzylamine

5 g (130 mmol) of lithium aluminum hydride was suspended in 300 ml of ethyl ether. After stirring at a room temperature, ether solution of 12 g (55 mmol) of 4-difluoromethylthioacetophenoneoxime was gradually dropwise added with stirring at a room temperature. After the dropwise addition was completed, the mixture was heated under reflux for 5 hours.

After the reaction, excess amount of the lithium aluminum hydride was decomposed with ethanol and subsequently water.

The insolubles were removed by filtration with Celite and the filtrate was extracted with 2N-hydrochloric acid. The extract was made alkaline with 2N-sodium hydroxide and extracted with ethyl acetate. After the extract was dried over anhydrous sodium sulfate, the solvent was removed by evaporation.

The resulting oily product was purified by silicagel column chromatography (Wako gel C-200, eluted with ethanol) to give 6 g of dl-α-methyl-4-difluoromethylthiobenzylamine which is a material compound (IIId).

Example 20

(1) Synthesis of dl-5-chloro-6-methyl-4-(α-methyl-4-difluoromethylthiobenzylamino)pyrimidine (Compound No. 74)

1.0 g (6.1 mmol) of 4,5-dichloro-6-methylpyrimidine which is a material compound (IIc), 1.0 g (4.9 mmol) of dl-α-methyl-4-difluoromethylthiobenzylamine which is a material compound (IIId), 1 ml of triethylamine were dissolved in 20 ml of toluene. The resulting solution was added with a catalytic amount of 4-(N,N-dimethylamino)pyridine and heated under reflux for 8 hours.

After the reaction, the reaction solution was washed with water were and dried over anhydrous sodium

sulfate, and the solvent was removed under reduced pressure.

The resulting oily product was isolated by Silicagel column chromatcgraphy (Wako gel C-200, eluted with toluene:ethyl acetate = 5:1) to give 0.9 g of the desired compound as colorless oily product (indicated in Table 23 as Compound No. 74).

(2) Synthesis of dl-5-chloro-6-ethyl-4-(α-methyl-4-difluoromethylthiobenzylamino)pyrimidine (Compound No. 79)

2.3 g (13 mmol) of 4,5-dichloro-6-ethylpyrimidine which is a material compound (IIc), 2.3 g (11 mmol) of dl-α-methyl-4-difluoromethylthiobenzylamine which is a material compound (IIId), 2 ml of trimethylamine were dissolved in ml of toluene. The resulting solution was added with a catalytic amount of 4-(N,N-dimethylamino)pyridine and heated under reflux for 8 hours.

After the reaction, the reaction solution was washed with water and dryed over anhydrous sodium sulfate.

The solvent was removed under reduced pressure. The resulting oily product was isolated by silicagel column chromatography (Wako gel C-200, eluted with toluene:ethyl acetate = 5:1). The resultant was crystallized with n-hexane to give 2.0 g of the desired compound as colorless crystals (indicated in Table 23 as Compound No. 79).

(3) Syntheses of dl-5-chloro-6-ethyl-4-(α-methyl-4-difluoromethanesulfinylbenzylamino)pyrimidine (Compound No. 80) and dl-5-chloro-6-ethyl-4-(α-methyl-4-difluoromethanesulfonylbenzylamino)pyrimidine (Compound No. 81)

2.6 g (7.6 mmol) of Compound No. 79 mentioned above was dissolved in 30 ml of methylene chloride. After stirring at a room temperature, 2.3 g (10.7 mmol) of m-chloro perbenzoic acid was added to the solution. After stirring for 5 hours, the reaction solution was extracted with chloroform. The extract was washed with 2N-sodium hydroxide solution and water and dried over anhydrous sodium sulfate. The solvent was removed by evaporation under reduced pressure and the resulting oily product was purified by silicagel column chromatography (Wako gel C-200, eluted with toluene:ethyl acetate = 3:1) to give 150 mg of the desired compound as colorless crystals (indicated in Table 23 as Compound No. 81) and 1.3 g of the desired compound as a colorless oily product (indicated in Table 23 as Compound No. 80).

(4) Syntheses of Compound Nos. 87 and 88 in Table 23

Desired compounds (Id-2) (indicated in Table 23 as Compound Nos. 87 and 88) were obtained according to any synthetic method as in the above (1) to (3).

Table 23

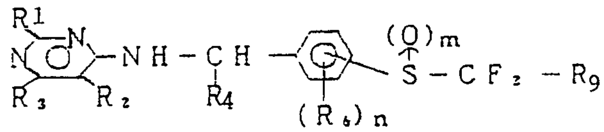 (Id-2)

| Compound | 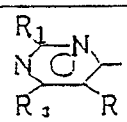 | R9 | R4 | $(R_6)n$ | $(O)_m$ $-SCF_2R_9$ substitution position | m | Physical property |
|---|---|---|---|---|---|---|---|
| 74 | 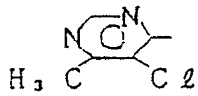 | H | $CH_3$ | H | 4 | 0 | $n_D^{21.8}$ 1.5888 |
| 75 | ″ | ″ | ″ | ″ | ″ | 1 | |
| 76 | ″ | ″ | ″ | ″ | ″ | 2 | |
| 77 | ″ | ″ | $C_2H_5$ | ″ | ″ | 0 | |
| 78 | ″ | ″ | ″ | ″ | 3 | ″ | |
| 79 | 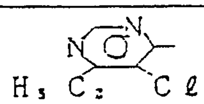 | ″ | $CH_3$ | ″ | 4 | ″ | m.p. 59~ 61 ℃ |
| 80 | ″ | ″ | ″ | ″ | ″ | 1 | $n_D^{24.6}$ 1.5837 |
| 81 | ″ | ″ | ″ | ″ | ″ | 2 | m.p. 114~116 ℃ |
| 82 | ″ | ″ | $C_2H_6$ | ″ | ″ | 0 | |
| 83 | ″ | ″ | ″ | ″ | 3 | ″ | |
| 84 | ″ | ″ | H | ″ | 4 | ″ | |
| 85 | ″ | ″ | $i\text{-}C_3H$ | ″ | ″ | ″ | |
| 86 | ″ | ″ | 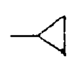 | ″ | ″ | ″ | |
| 87 | 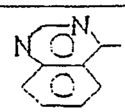 | ″ | $CH_3$ | ″ | ″ | ″ | m.p. 73~ 76 ℃ |
| 88 | 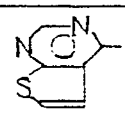 | ″ | ″ | ″ | ″ | ″ | m.p. 155~158 ℃ |

Example 21

(1) Preparation of granules

5 parts by weight of Compound No. 74, 35 parts by weight of bentonite, 57 parts by weight of talc, 1 part by weight of Neopelex powder (trade name; manufactured by Kao Atlas) and 2 parts by weight of sodium ligninsulfonate were uniformly mixed, and then kneaded with addition of a small amount of water, followed by granulation and drying to obtain granules.

(2) Preparation of wettable powder

50 parts by weight of Compound No. 74, 48 parts by weight of kaolin and 2 parts by weight of

Neopelex powder (trade name; manufactured by Kao Atlas) were uniformly mixed, followed by pulverization to obtain wettable powder.

(3) Preparation of emulsion

To 20 parts by weight of Compound No. 74 and 70 parts by weight of xylene were added 10 parts by weight of Toxanone (trade name; manufactured by Sanyo Kasei Kogyo), and the mixture was uniformly mixed and dissolved to obtain an emulsion.

(4) Preparation of powder

5 parts by weight of Compound No. 74, 50 parts by weight of talc and 45 parts by weight of kaolin were uniformly mixed to obtain powder.

Example 22

(1) Control activity test (preventive activity) against wheat brown rust

In plastic planting pots of 6 cm in diameter, ten wheats (species: Kobushibomugi) were grown per each pot, and to young plants at the 1.5 leaf stage were sprayed the wettable powder of the respective compounds of the desired compound (Id-2) shown in Table 23, prepared similarly as in Example 21, diluted to 50 ppm with water containing a surfactant (0.01 %) in an amount of 20 ml per each pot.

After spraying, the plants were cultivated in a glass house for 2 days, and then a wheat brown rust Puccinia recondita ) spore suspension (7 x $10^4$ spores/ml) was uniformly inoculated by spraying thereon.

After inoculation, the plants were grown in a glass house for one week, and the extent of the wheat brown rust lesion appeared on the first leaf was examined.

Evaluation is shown by the 6 ranks of 5 to 0 judging by comparison with the extent of deseases of the non-treated group. In the standard of the comparison, one which is wholly afflicted with the disease as 0, one with lesion area of about 60 % as compared with the non-treated group as 1, about 40 % as 2, about 20 % as 3, about 10 % as 4, and one without lesion being rated as 5. The results are shown in Table 24.

As control, the following compound A disclosed in Japanese Unexamined Patent Publication No. 35555/1984 and compound B disclosed in Japanese Unexamined Patent Publication No. 68362/1989.

Compound A

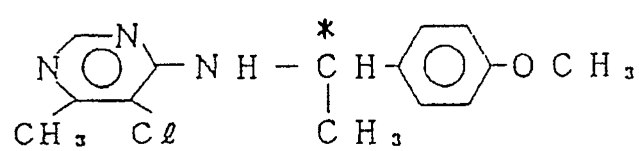

Compound B

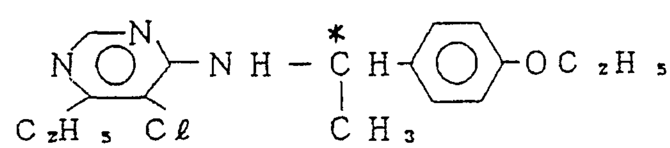

Table 24

| Compound No. | Preventive activity against wheat brown rust |
|---|---|
| 74 | 5 |
| 79 | 5 |
| 80 | 5 |
| 81 | 4 |
| Compound A | 1 |
| Compound B | 1 |
| No treatment | 0 |

(2) Control activity test (preventive activity) against barley powdery mildew

In plastic planting pots of 6 cm in diameter, ten barleys (species: Black barley) were grown per each pot, and to young plants at the 1.5 leaf stage was sprayed the wettable powder of the respective compounds of the desired compound (Id-2) as shown in Table 23, prepared similarly as in Example 21, diluted to 50 ppm with water containing a surfactant (0.01 %) in an amount of 20 ml per each pot.

After spraying, the plants were cultivated in a glass house for 2 days, and then conidial spores of barley powdery mildew (Erysiphe graminis ) were collected from the afflicted leaves and sprayed uniformly on the plants to effect inoculation.

After inoculation, the plants were grown in a glass house for one week, and the extent of the barley powdery mildew lesion appeared on the first leaf was examined.

The results of evaluation for drug effect were judged by the same evaluation method as in the above (1) and are shown in Table 25. As a control, the same compounds A and B as in the above (1) were used.

Table 25

| Compound No. | Preventive effect against barley powdery mildew |
|---|---|
| 74 | 5 |
| 79 | 5 |
| 80 | 5 |
| Compound A | 1 |
| Compound B | 1 |
| No treatment | 0 |

(3) Control activity test (preventive activity) against cucumber downy mildew

In plastic planting pots of 6 cm in diameter, one cucumber (species: Sagamihanjiro) was grown per each pot, and to young plants at the 1.5 leaf stage was sprayed the wettable powder of the desired compound (Id-2) as shown in Table 23, prepared similarly as in Example 21, diluted to 500 ppm with water containing a surfactant (0.01 %) in an amount of ml per each pot.

After spraying, the plants were cultivated in a glass house for 2 days, and then cucumber downy mildew (Pseudoperonospora cubensis ) zoosporangia were prepared from the afflicted leaves and sprayed uniformly on the plants to effect inoculation.

After inoculation, the plants were maintained under dark at 20 °C for 2 days and grown in a glass house for five days, and the extent of the cucumber downy mildew lesion appeared on the first leave was

examined.

The drug effect was judged under the same evaluation method as in the above (1) and the results are shown in Table 26.

As a control, the same compounds A and B as in the above (1) were used.

Table 26

| Compound No. | Preventive activity against cucumber downy mildew |
|---|---|
| 74 | 5 |
| 79 | 5 |
| 80 | 5 |
| 81 | 5 |
| 88 | 5 |
| Compound A | 0 |
| Compound B | 0 |
| No treatment | 0 |

Example 23

[Preparation of amine represented by the formula (VIII)]

(1) Preparation of 3,4-(difluoromethylenedioxy)benzylamine

0.5 g (13 mmol) of lithium aluminum hydride was suspended in 10 ml of anhydrous ethyl ether. To the resulting solution was gradually added dropwise a solution of 1 g (5.5 mmol) of 3,4-(difluoromethylenedioxy)benzonitrile dissolved in anhydrous ethyl ether. The solution was heated under reflux for 2 hours. After the reaction, excess amount of lithium-aluminum hydride was decomposed with ethanol and subsequently water, and extracted with ethyl ether. After drying over anhydrous sodium sulfate, the solvent was removed by evaporation under reduced pressure. The resultant was purified by column chromatography to obtain 0.5 g of 3,4-(difluoromethylenedioxy)benzylamine which is colorless oily products.
CI-Mass (m/e), 188 ($M^+$ +1), 171 ($M^+$ -16)

(2) Preparation of α-ethyl-3,4-(difluoromethylenedioxy)benzylamine

1.3 g (5.5 mmol) of 3,4-(difluoromethylenedioxy)propiophenone oxime and 1 g of Raney nickel were suspended in 50 ml of ethanol, and the suspension was saturated by ammonia gas. The mixture was stirred in an autoclave under a hydrogen pressure of 30 kg/cm$^2$ at 100 °C for 6 hours. After the reaction, insolubles were removed by filtration and the solvent was removed by evaporation. The resultant was purified by column chromatography to obtain 1 g of α-ethyl-3,4-(difluoromethylenedioxy)benzylamine which is colorless oily products.
CI-Mass (m/e), 216 ($M^+$ +1), 199 ($M^+$ -16), 186 (M+ -29)

(3) Preparation of α-(2,2,3-trifluorobenzo-1,4-dioxane-6-yl)ethylamine

6.7 g of 6-(α-hydroxyiminoethyl)-2,2,3-trifluorobenzo-1,4-dioxane and 4 g of Raney nickel were suspended in 70 ml of ethanol, and the suspension was saturated with ammonia gas. The mixture was stirred in an autoclave under a hydrogen pressure of 30 kg/cm$^2$ at 100 °C for 6 hours. After the reaction, insolubles were removed by filtration and the solvent was removed by evaporation. The resultant was purified by column chromatography to obtain 5.5 g of α-(2,2,3-trifluorobenzo-1,4-dioxane-6-yl)ethylamine,

which is colorless oily products.
CI-Mass (m/e), 233 ($M^+$ +1), 217 ($M^+$ -15)

Example 24

[Preparation of oxime represented by the formula (IX)]

(1) Preparation of 3,4-(difluoromethylenedioxy)propiophenone oxime

1.5 g (7 mmol) of 3,4-(difluoromethylenedioxy)propiophenone, 1.5 g (22 mmol) of hydroxylamine hydrochloride and 1.5 g (36 mmol) of sodium hydroxide were dissolved in a mixture of ethanol 15 ml-water 5 ml, and the solution was stirred at 70 °C for 3 hours. After the reaction, the solvent was removed by evaporation and extraction with ethyl acetate and washing with water were conducted. After drying over anhydrous sodium sulfate, the solvent was removed by evaporation under reduced pressure. The settled crystals were washed with hexane to obtain 1.3 g of 3,4-(difluoromethylenedioxy)propiophenone oxime which is colorless crystals.

CI-Mass (m/e), 230 ($M^+$ +1), 212 ($M^+$ -17), 184 ($M^+$ -55)

(2) Preparation of 6-(α-hydroxyiminoethyl)-2,2,3-trifluorobenzo-1,4-dioxane

7 g (30 mmol) of 6-acetyl-2,2,3-trifluorobenzo-1,4-dioxane, 3 g (43 mmol) of hydroxylamine hydrochloride and 1.7 g (43 mmol) of sodium hydroxide were dissolved in a mixture of ethanol 50 ml-water 10 ml, and the solution was stirred at 70 °C for 3 hours. After the reaction, the solvent was removed by evaporation, followed by extraction with ethyl acetate and washing with water. After drying over anhydrous sodium sulfate, the solvent was removed by evaporation under reduced pressure. The settled crystal was washed with water to obtain 6.5 g of 6-(α-hydroxyiminoethyl)2,2,3-trifluorobenzo-1,4-dioxane, which is colorless crystals.
CI-Mass (m/e), 248 ($M^+$ +1), 230 (M+ -17)

Example 25

[Preparation of ketone represented by the formula (X)]

(1) Preparation of 3,4-(difluoromethylenedioxy)propiophenone

To a solution of ethylmagnesium bromide dissolved in ether (EtMgBr: 12 mmol) were gradually added dropwise a solution of 1.2 g of 3,4-(difluoromethylenedioxy)benzonitrile dissolved in 8 ml of anhydrous ether. After heating under reflux for 2 hours, the reaction solution was poured into ice-cooling water, followed by extraction with ethyl acetate and washing with water. After drying over anhydrous sodium sulfate, the solvent was removed by evaporation under reduced pressure to obtain 1.4 g of 3,4-(difluoromethylenedioxy)propiophenone which is colorless oily products.
CI-Mass (m/e), 215 ($M^+$ +1), 185 ($M^+$ -29)

(2) Preparation of 6-acetyl-2,2,3-trifluorobenzo-1,4-dioxane

10 g (66 mmol) of 3,4-dihydroxyacetophenone and 4.4 g (78 mmol) of potassium hydroxide powder were suspended in 30 ml of sulforane. The suspension was heated to 90 °C under nitrogen atomosphere.

Trifluorochloroethylene was brown therethrough at a temperature of 100 to 110 °C until the starting material, 3,4-dihydroxyacetophenone, is completely consumed. After cooling of the reaction solution, extraction with toluene and washing with water were conducted. After drying over anhydrous sodium sulfate, the solvent was removed by filtration. The resulting oily product was purified by column chromatography (Wako Gel C200, eluted with toluene:ethyl acetate = 10:1) to obtain 7 g of 6-acetyl-2,2,3-trifluorobenzo-1,4-dioxane, which is colorless oily products.

CI-Mass (m/e), 233 ($M^+$ +1), 218 ($M^+$ -15)

As shown in the above, the compounds having fluoromethylthio group of the present invention have remarkably improved fungicidal effect as compared with analogous compounds having methoxy group.

## Claims

1. An aralkylamine derivative represented by the following formula (I) and acid addition salt thereof:

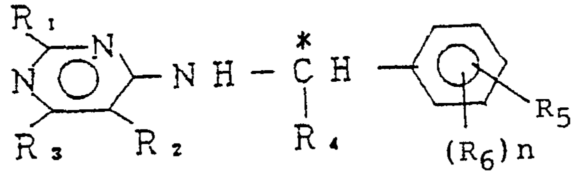

(I)

wherein $R_1$ represents hydrogen atom, a halogen atom, a lower alkyl group, a cycloalkyl group, a lower alkoxy group, a halo-lower alkyl group, an amino group which may be substituted with a lower alkyl group, or a phenyl group which may be substituted;

$R_2$ and $R_3$ each represent hydrogen atom, a halogen atom or a lower alkyl group, or $R_2$ and $R_3$ are fused to the pyrimidine ring together with carbon atoms to which they are bonded to represent a saturated or unsaturated 5- or 6-membered ring which may also have one sulfur atom;

$R_4$ represents hydrogen atom, a lower alkyl group, a cycloalkyl group or a halo-lower alkyl group;

$R_5$ represents -O-$A_1$-CN or

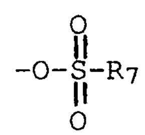

at the 4th position, where $A_1$ represents a lower alkylene group, $R_7$ represents a lower alkyl group, a halo-lower alkyl group; a phenyl group, a benzyl group or a styryl group which may have a substituent; or an amino group which may be substituted with a lower alkyl group; in this case, $R_6$ represents hydrogen atom; or $R_5$ represents

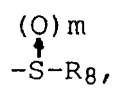

where $R_8$ represents -$CF_2$-$R_9$ (wherein $R_9$ represents hydrogen atom or a halogen atom) or an alkyl group which is substituted with a lower alkoxy group or a lower alkylthio group; in this case, $R_6$ represents hydrogen atom, a halogen atom, a lower alkyl group, a lower alkoxy group or a halo-lower alkyl group;

or $R_5$ and $R_6$ may form -O-$A_2$-O- at the 3- and 4-positions, where $A_2$ represents methylene or ethylene group substituted with 1 to 4 halogen atoms;

m represents 0, 1 or 2; and

n represents 1 or 2.

2. The aralkylamine derivative and acid addition salt thereof according to Claim 1, wherein the $R_1$ is at least one selected from the group consisting of hydrogen atom, chlorine atom and methyl group.

3. The aralkylamine derivative and acid addition salt thereof according to Claim 1, wherein the $R_1$ is hydrogen atom.

4. The aralkylamine derivative and acid addition salt thereof according to Claim 1, wherein the $R_2$ is a halogen atom.
5. The aralkylamine derivative and acid addition salt thereof according to Claim 1, wherein the $R_2$ is chlorine atom.
6. The aralkylamine derivative and acid addition salt thereof according to Claim 1, wherein the $R_3$ is a lower alkyl group.
7. The aralkylamine derivative and acid addition salt thereof according to Claim 1, wherein the $R_3$ is at least one selected from the group consisting of methyl group, ethyl group and isopropyl group.
8. The aralkylamine derivative and acid addition salt thereof according to Claim 1, wherein the $R_2$ and $R_3$ are fused to form a benzene ring or a thiophene ring.
9. The aralkylamine derivative and acid addition salt thereof according to Claim 1, wherein the $R_4$ is selected from the group consisting of hydrogen atom, a lower alkyl group and a cycloalkyl group.
10. The aralkylamine derivative and acid addition salt thereof according to Claim 1, wherein the $R_4$ is selected from the group consisting of hydrogen atom, a straight- or branched-chain alkyl group having 1 to 8 carbon atoms and a cycloalkyl group having 3 to 8 carbon atoms.
11. The aralkylamine derivative and acid addition salt thereof according to Claim 1, wherein the $R_4$ is selected from the group consisting of a straight- or branched-chain alkyl group having 1 to 3 carbon atoms and cycloalkyl group having 3 to 6 carbon atoms.
12. The aralkylamine derivative and acid addition salt thereof according to Claim 1, wherein the $R_4$ is selected from the group consisting of methyl group, ethyl group, isopropyl group, cyclopropyl group, cyclopentyl group and cyclohexyl group.
13. The aralkylamine derivative and acid addition salt thereof according to Claim 1, wherein the $R_6$ is hydrogen atom.
14. The aralkylamine derivative and acid addition salt thereof according to Claim 1, wherein the $R_7$ is at least one selected from the group consisting of methyl, ethyl, propyl, isopropyl, butyl, sec-butyl, t-butyl, pentyl, isopentyl, neopentyl, monofluoromethyl, difluoromethyl, trifluoromethyl, monochloromethyl, trichloromethyl, 2-fluoroethyl, 2,2,2-trifluoroethyl, 1,1,2,2,2-pentafluoroethyl, 3,3,3-trifluoropropyl, 3-chloropropyl, phenyl, 4-chlorophenyl, 4-fluorophenyl, 4-methylphenyl, 4-trifluoromethylphenyl, amino, monomethylamino, dimethylamino, monoethylamino, diethylamino, monopropylamino, dipropylamino, monobutylamino, dibutylamino, monopentylamino and dipentylamino groups.
15. The aralkylamine derivative and acid addition salt thereof according to Claim 1, wherein the $R_8$ is at least one selected from the group consisting of methyl group, ethyl group and 2-ethoxyethyl group.
16. The aralkylamine derivative and acid addition salt thereof according to Claim 1, wherein the $R_9$ is hydrogen atom.
17. The aralkylamine derivative and acid addition salt thereof according to Claim 1, wherein the lower alkylene group as $A_1$ is at least one selected from the group consisting of methylene, 1-methylmethylene, ethylene, 1-methylethylene, 2-methylethylene, propylene and pentylene groups.
18. The aralkylamine derivative and acid addition salt thereof according to Claim 1, wherein the lower alkylene group as $A_1$ is at least one selected from the group consisting of methylene group, 1-methylmethylene group, ethylene group and propylene group.
19. The aralkylamine derivative and acid addition salt thereof according to Claim 1, wherein the lower alkylene group as $A_1$ is at least one selected from the group consisting of methylene group, ethylene group and propylene group.
20. The aralkylamine derivative and acid addition salt thereof according to Claim 1, wherein the $A_2$ is at least one selected from the group consisting of monofluoromethylene group, difluoromethylene group, monofluoroethylene group, difluoroethylene group, trifluoroethylene group and tetrafluoroethylene group.
21. The aralkylamine derivative and acid addition salt thereof according to Claim 1, wherein the aralkylamine derivative is a compound represented by the formula (Ia) shown below:

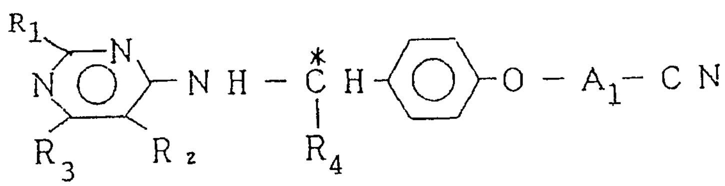

(Ia)

(wherein $R_1$, $R_2$, $R_3$, $R_4$ and $A_1$ have the same meanings as defined in Claim 1).
22. The aralkylamine derivative and acid addition salt thereof according to Claim 1, wherein the aralkylamine derivative is a compound represented by the formula (Ib) shown below:

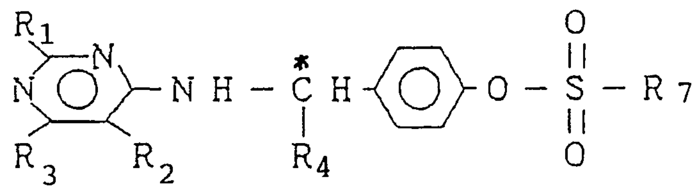

(Ib)

(wherein $R_1$, $R_2$, $R_3$, $R_4$ and $R_7$ have the same meanings as defined in Claim 1).

23. The aralkylamine derivative and acid addition salt thereof according to Claim 1, wherein the aralkylamine derivative is a compound represented by the formula (Ic) shown below:

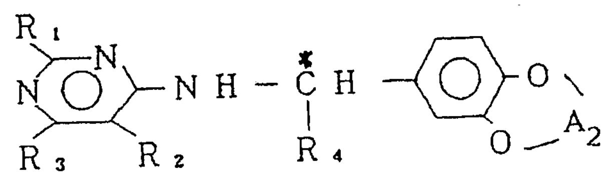

(Ic)

(wherein $R_1$, $R_2$, $R_3$, $R_4$ and $A_2$ have the same meanings as defined in Claim 1).

24. The aralkylamine derivative and acid addition salt thereof according to Claim 1, wherein the aralkylamine derivative is a compound represented by the formula (Id′) shown below:

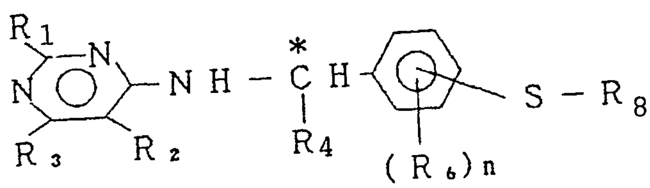

(Id')

(wherein $R_1$, $R_2$, $R_3$, $R_4$, $R_6$, $R_8$ and n have the same meanings as defined in Claim 1).

25. The aralkylamine derivative and acid addition salt thereof according to Claim 1, wherein the aralkylamine derivative is a compound represented by the formula (Id″) shown below:

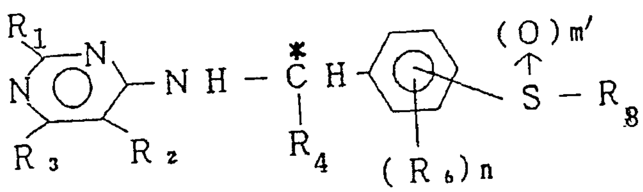

(Id")

(wherein $R_1$, $R_2$, $R_3$, $R_4$, $R_6$, $R_8$ and n have the same meanings as defined in Claim 1 and m′ represents 1 or 2).

26. A method for producing a compound represented by the formula (I):

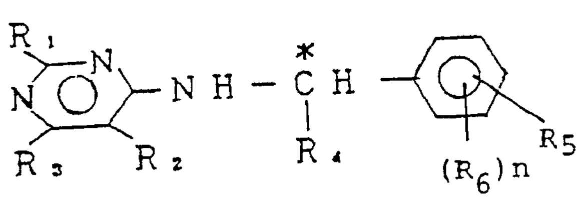

(I)

(wherein $R_1$, $R_2$, $R_3$, $R_4$, $R_5$ ,$R_6$ and n have the same meanings as defined in Claim 1), which comprises reacting a compound represented by the formula (IIa):

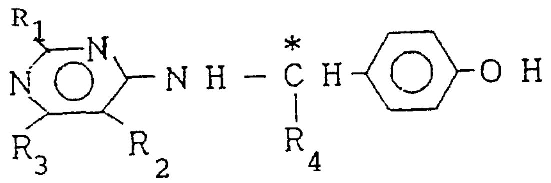

(IIa)

(wherein $R_1$, $R_2$, $R_3$ and $R_4$ have the same meanings as defined above), with at least one of a compound represented by the formula (IIIa):

X-$A_1$-CN (IIIa)

and a compound represented by the formula (IIIb):

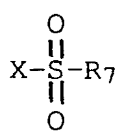

(IIIb)

(wherein $A_1$ and $R_7$ the same meaning as defined above and X represents an eliminable group).

27. The method for producing the compound of the formula (I) according to Claim 26, wherein the eliminable group represented by X is at least one selected from the group consisting of a halogen atom, an alkylthio group, an alkanesulfonyloxy group which may be substituted with a halogen atom, an arylsulfonyloxy group and hydroxy group.

28. The method for producing the compound of the formula (I) according to Claim 26, wherein the eliminable group represented by X is at least one selected from the group consisting of chlorine atom, bromine atom, iodine atom and methylthio, ethylthio, propylthio, butylthio, methanesulfonyloxy, ethanesulfonyloxy, trifluoromethanesulfonyloxy, benzenesulfonyloxy, p-toluenesulfonyloxy and hydroxy groups.

29. The method for producing the compound of the formula (I) according to Claim 26, wherein the eliminable group represented by X is halogen atom.

30. The method for producing the compound of the formula (I) according to Claim 26, wherein the eliminable group represented by X is chlorine atom.

31. A method for producing a compound represented by the formula (Ic) and acid addition salt thereof:

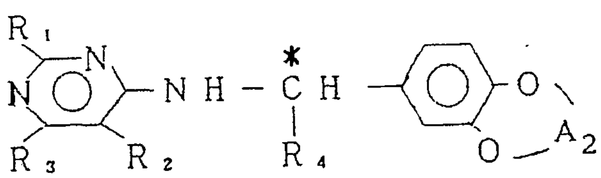

(Ic)

(wherein $R_1$, $R_2$, $R_3$, $R_4$, $A_2$ and n have the same meanings as defined in Claim 1), which comprises reacting a compound represented by the formula (IIb):

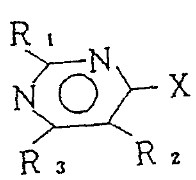

(IIb)

(wherein $R_1$, $R_2$, $R_3$ and X have the same meanings as defined above), with a compound represented by the formula (IIIc):

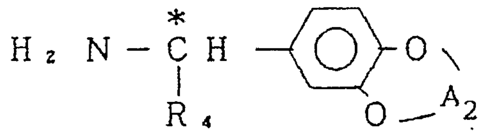

(IIIc)

(wherein $R_4$ and $A_2$ have the same meaning as defined above).

32. A method for producing aralkylamine derivatives or acid addition salts thereof represented which comprises:

reacting a compound represented by the formula (IIc):

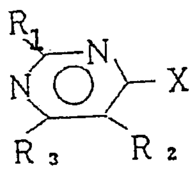

(IIc)

(wherein $R_1$, $R_2$, $R_3$ and X have the same meanings as defined above), with a compound represented by the formula (IIId):

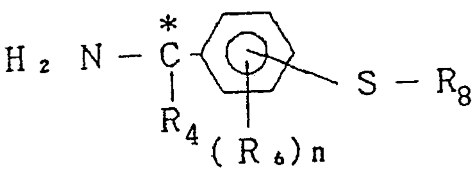

(IIId)

(wherein $R_4$, $R_6$, $R_8$ and n have the same meaning as defined above) to obtain a compound represented by the formula (I-d′):

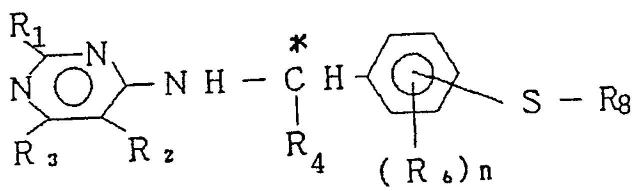

(I-d')

(wherein $R_1$, $R_2$, $R_3$, $R_4$, $R_6$, $R_8$ and n have the same meanings as defined above),

and then optionally oxidizing the compound represented by the formula (I-d′) with a peroxide to obtain a compound represented by the formula (I-d″)

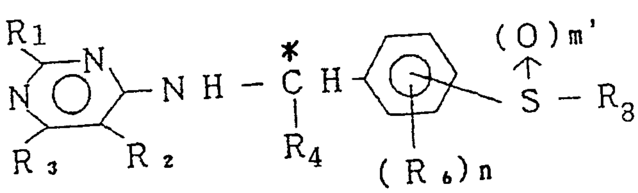

(I-d")

(wherein $R_1$, $R_2$, $R_3$, $R_4$, $R_6$, $R_8$ and n have the same meanings as defined above and m′ represents 1 or 2)

33. A fungicide containing the aralkylamine derivatives or acid addition salts thereof as claimed in Claim 1 as an active ingredient.

Claims for the following Contracting State:ES

1. A process for preparing a compound represented by the formula (I), and acid addition salts thereof:

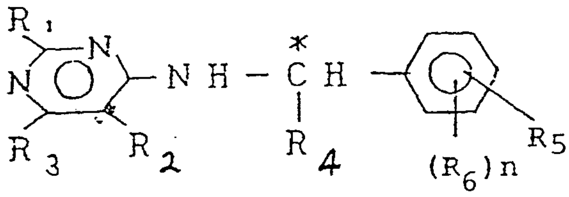

(I)

wherein $R_1$ represents hydrogen atom, a halogen atom, a lower alkyl group, a cycloalkyl group, a lower alkoxy group, a halo-lower alkyl group, an amino group which may be substitued with a lower alkyl group, or a phenyl group which may be substituted;
$R_2$ and $R_3$ each represent hydrogen atom, a halogen atom or a lower alkyl group, or $R_2$ and $R_3$ are fused to the pyrimidine ring together with carbon atoms to which they are bonded to represent a saturated or unsaturated 5- or 6-membered ring which may also have one sulfur atom;
$R_4$ represents hydrogen atom, a lower alkyl group, a cycloalkyl group or a halo-lower alkyl group;
$R_5$ represents $-O-A_1-CN$ or

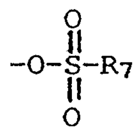

at the 4th position,
where $A_1$ represents a lower alkylene group, $R_7$ represents a lower alkyl group, a halo-lower alkyl group; a phenyl group, a benzyl group or a styryl group which may have a substituent; or an amino group which may be substituted with a lower alkyl group; in this case, $R_6$ represents hydrogen atom;
or $R_5$ represents

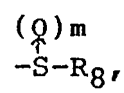

where $R_8$ represents $-CF_2-R_9$ (wherein $R_9$ represents hydrogen atom or a halogen atom) or an alkyl group which is substituted with a lower alkoxy group or a lower alkylthio group; in this case, $R_6$ represents hydrogen atom, a halogen atom, a lower alkyl group, a lower alkoxy group or a halo-lower alkyl group;
or $R_5$ and $R_6$ may form $-O-A_2-O-$ at the 3- and 4-positions, where $A_2$ represents methylene or ethylene group substituted with 1 to 4 halogen atoms;
m represents 0, 1 or 2; and
n represents 1 or 2,
which comprises reacting a compound reprented by the formula (IIa):

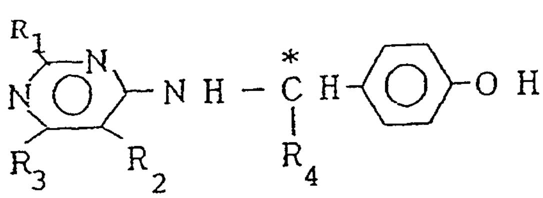

(IIa)

(wherein $R_1$, $R_2$, $R_3$ and $R_4$ have the same meanings as defined above), with a compound represented by the formula (IIIa):
$X-A_1-CN$ (IIIa)
or a compound represented by the formula (IIIb):

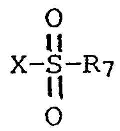

(IIIb)

(wherein $A_1$ and $R_7$ have the same meaning as defined above and X represents an eliminable group).

2. A process as claimed in Claim 1, wherein $R_1$ is a hydrogen atom, chlorine atom or methyl group.

3. A process as claimed in Claim 1, wherein $R_1$ is a hydrogen atom.

4. A process as claimed in any one of Claims 1 to 3, wherein $R_2$ is a halogen atom.

5. A process as claimed in any one of Claims 1 to 3, wherein $R_2$ is a chlorine atom.

6. A process as claimed in any one of Claims 1 to 5, wherein $R_3$ is a lower alkyl group.

7. A process as claimed in any one of Claims 1 to 5, wherein $R_3$ is a methyl group, ethyl group or isopropyl group.

8. A process as claimed in any one of Claims 1 to 3, wherein $R_2$ and $R_3$ are fused to form a benzene ring or a thiophene ring.

9. A process as claimed in any one of Claims 1 to 8, wherein $R_4$ is a hydrogen atom, a lower alkyl group or a cycloalkyl group.

10. A process as claimed in any one of Claims 1 to 8, wherein $R_4$ is a hydrogen atom, a straight- or branched-chain alkyl group having 1 to 8 carbon atoms or a cycloalkyl group having 3 to 8 carbon atoms.

11. A process as claimed in any one of Claims 1 to 8, wherein $R_4$ is a straight- or branched-chain alkyl group having 1 to 3 carbon atoms or a cycloalkyl group having 3 to 6 carbon atoms.

12. A process as claimed in any one of Claims 1 to 8, wherein $R_4$ is a methyl, ethyl, isopropyl, cyclopropyl, cyclopentyl or cyclohexyl group.

13. A process as claimed in any. one of Claims 1 to 13, wherein $R_6$ is a hydrogen atom.

14. A process as claimed in any one of Claims 1 to 3, wherein $R_7$ is a methyl, ethyl, propyl, isopropyl, butyl, sec-butyl, t-butyl, pentyl, isopentyl, neopentyl, monofluoromethyl, difluoromethyl, trifluoromethyl, monochloromethyl, trichloromethyl, 2-fluoroethyl, 2,2,2-trifluoroethyl, 1,1,2,2,2-pentafluoroethyl, 3,3,3-trifluoropropyl, 3-chloropropyl, phenyl, 4-chlorophenyl, 4-fluorophenyl, 4-methylphenyl, 4-trifluoromethylphenyl, amino, monomethylamino, dimethylamino, monoethylamino, diethylamino, minonopropylamino, dipropylamino, monobutylamino, dibutylamino, monopentylamino or dipentylamino group.

15. A process as claimed in any one of Claims 1 to 14, wherein $R_8$ is a methyl group, ethyl group or 2-ethoxyethyl group.

16. A process as claimed in any one of Claims 1 to 15, wherein $R_9$ is a hydrogen atom.

17. A process as claimed in any one of Claims 1 to 16, wherein the lower alkylene group $A_1$ is a methylene, 1-methylmethylene, ethylene, 1-methylethylene, 2-methylethylene, propylene or pentylene group.

18. A process as claimed in any one of Claims 1 to 17, wherein $A_2$ is a monofluoromethylene group, difluoromethylene group, monofluoroethylene group, difluoroethylene group, trifluoroethylene group or tetrafluoroethylene group.

19. A process as claimed in any one of Claims 1 to 18, wherein the eliminable group represented by X is a halogen atom, an alkylthio group, an alkanesulfonyloxy group which may be substituted with a halogen atom, an arylsulfonyloxy group or a hydroxy group.

20. A process as claimed in any one of Claims 1 to 18, wherein the eliminable group represented by X is a chlorine atom, bromine atom, iodine atom, methylthio, ethylthio, propylthio, butylthio, methanesulfonyloxy, ethanesulfonyloxy,trifluoromethanesulfonyloxy, benzenesulfonyloxy, p-toluenesulfonyloxy or hydroxy group.

21. A process as claimed in any one of Claims 1 to 18, wherein the eliminable group represented by X is a halogen atom.

22. A process as claimed in any one of Claims 1 to 18, wherein the eliminable group represented by X is a chlorine atom.

23. A process for preparing a compound represented by the formula (Ic) and acid addition salts thereof:

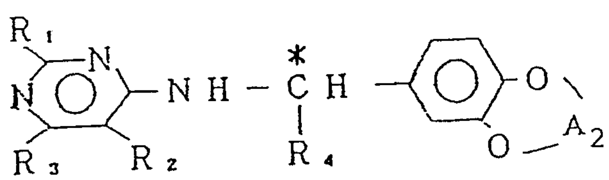

(Ic)

(wherein $R_1$, $R_2$, $R_3$, $R_4$, $A_2$ and n have the same meaning as defined in Claim 1), which comprises reacting a compound represented by the formula (IIb):

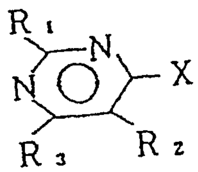

(IIb)

(wherein $R_1$, $R_2$, $R_3$ and X have the same meaning as defined in Claim 1 with a compound represented by the formula (IIIc):

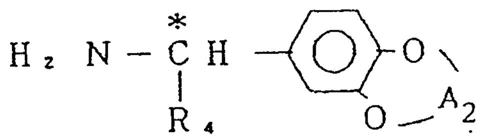

(IIIc)

(wherein $R_4$ and $A_2$ have the same meaning as defined in Claim 1.

24. A process for preparing aralkylamine derivatives or acid addition salts thereof which comprises reacting a compound represented by the formula (IIc):

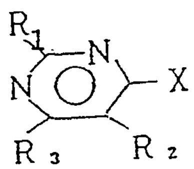

(IIc)

(wherein $R_1$, $R_2$, $R_3$ and X have the same meanings as defined in Claim 1 with a compound represented by the formula (IIId):

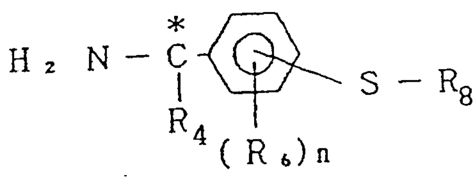

(IIId)

(wherein $R_4$, $R_5$, $R_8$ and n have the same meaning as defined in Claim 1 to obtain a compound represented by the formula (I-d′):

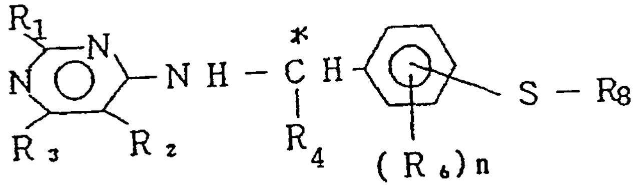

(I-d')

and then optionally oxidizing the compound represented by the formula (I-d′) with a peroxide to obtain a compound represented by the formula (I-d″):

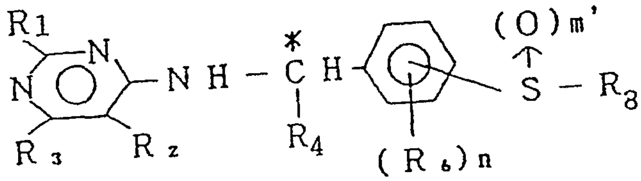

(I-d")

(wherein m represents 1 or 2).

25. A fungicide containing the aralkylamine derivatives or acid addition salts thereof of formula (I) as defined in Claim 1 as active ingredient.